# EUROPÄISCHE PATENTSCHRIFT

(11) **EP 2 838 437 B1**
(45) Veröffentlichungstag und Bekanntmachung des Hinweises auf die Patenterteilung: **07.09.2016**
(21) Anmeldenummer: 14723022.1
(22) Anmeldetag: 24.04.2014
(51) Int. Cl.: A61B 17/02, A61B 90/50

(54) **TELESKOPIERBARE RETRAKTORHALTERUNG**
TELESCOPING RETRACTOR HOLDER
ÉLÉMENT DE RETENUE TÉLESCOPIQUE D'ÉCARTEUR

(30) Priorität: 26.04.2013 DE 102013104300
(43) Veröffentlichungstag der Anmeldung: 25.02.2015
(73) Patentinhaber: Aesculap AG, 78532 Tuttlingen (DE)
(72) Erfinder: VOGTHERR, Robert, 78532 Tuttlingen (DE); BECK, Thomas, 78591 Durchhausen (DE); MORALES, Pedro, 78532 Tuttlingen (DE); WEISSHAUPT, Dieter, 78194 Immendingen (DE)
(74) Vertreter: Winter, Brandl, Fürniss, Hübner, Röss, Kaiser, Polte - Partnerschaft mbB
(86) Internationale Anmeldenummer: PCT/EP2014/058377
(87) Internationale Veröffentlichungsnummer: WO 2014/174031

(56) Entgegenhaltungen:
- WO-A1-2013/060581
- DE-U1- 29 804 127
- US-A- 3 710 783
- US-A- 4 380 999
- US-A- 5 000 163
- US-A- 5 573 495
- US-A- 5 882 299
- US-A1- 2010 087 819
- US-B2- 7 887 481

## Beschreibung

Die vorliegende Erfindung betrifft eine Teleskopierbare Retraktorhalterung und genauer gesagt eine chirurgische Hub-Vorrichtung mit Einhand-Bedienung zum Anheben eines Retraktors zum Beispiel zur Mammaria-Präparation.

### Hintergrund der Erfindung

Bei vielen herzchirurgischen Eingriffen muss das Sternum des Patienten in Längsrichtung durchtrennt und anschließend die Sternumshälften mit den daran anschließenden Rippen auseinander gespreizt werden, um dem Operateur das Arbeiten am Herz zu ermöglichen. Zum Spreizen dieser Operationsöffnung werden Retraktoren eingesetzt. Wenn bei dem Eingriff Durchblutungsstörungen der Herzkranzgefäße behandelt werden sollen, wird eine so genannte Bypass-Operation durchgeführt. Eine der am weitest verbreiteten Operationsmethoden ist die Schaffung eines so genannten Arteria-Mammaria-Bypasses. Bei derartigen Bypass-Operationen muss ein Abschnitt einer verengten Koronararterie umgangen werden. Die Arteria Mammaria Interna (IMA), welche hierbei als Bypass-Gefäß verwendet wird, verläuft längs an der Innenseite des Brustkorbs entlang. Ein Stück dieser Arterie wird vom Operateur freipräpariert und das Ende an eine Koronararterie des Herzens angenäht.

Normalerweise wird die linke Brustwandarterie (LIMA) verwendet, häufig allerdings auch zusätzlich die rechte Arterie (RIMA). Um dem Operateur für diese Arbeiten genügend Arbeitsraum und ausreichende Sichtverhältnisse bereitstellen zu können, wird ein Hub-System benötigt, das eine Hälfte des durchtrennten Sternums mit den sich daran anschließenden Rippen in vertikaler Richtung anheben und abstützen kann.

### Stand der Technik

Im Stand der Technik sind verschiedenste Systeme dieser Gattung bekannt. Eine erste Art von Hub- Systemen, die beispielsweise in den Patentschriften EP 0 931 509 B1 oder US 6.416.468 B2 offenbart ist, basiert auf einem gewöhnlichen Retraktor mit einer Adaptervorrichtung, die am Retraktor angebaut wird und ihn einseitig anhebt. In der Patentschrift EP 0 931 509 B1 wird das Anheben mit einer Schraube mit Abstütz-Teller und einem schwenkbar am Retraktor angebrachten Gewinde realisiert. Das Hub-System wird an den Retraktor geklemmt oder geschraubt. Gemäß der Patentschrift US 6.416.468 B2 werden die Schraube und das Gewinde durch einen Rastmechanismus ersetzt, die prinzipielle Funktionsweise unterscheidet sich jedoch nicht. Das Anheben einer Seite des Sternums geschieht, indem der Abstütz-Teller gegen die Rippen gedrückt wird und somit die Seite des Sternums, auf der sich das Hub-System befindet, nach außen gezogen wird.

Sehr ähnlich ist die zweite Art von Hub-Systemen, die mit speziellen Valven an einem ansonsten ebenfalls gewöhnlichen Retraktor arbeiten. Das Prinzip des oben beschriebenen Anhebens wird hier anstatt über eine Adaptervorrichtung über eine spezielle Valve, welche gelenkig am Retraktorrahmen angebracht ist, und ein Abstandselement erreicht. Mit Hilfe des Abstandselements wird der Abstand der schwenkbaren Seite der speziellen Valve zu dem Retraktorrahmen eingestellt und somit die Anhebung der entsprechenden Sternumshälfte, Derartige Systeme sind in den Dokumenten US 5,025,779 A und DE 10 325 393 B3 beschrieben.

Aus der US 3,710,783 ist ein weiteres Hub-System bekannt.

Bekannt sind auch reine Mammaria-Retraktoren, die nicht für das normale Sternum-Spreizen, sondern nur für das Durchführen der Präparation der Arteria Mammaria verwendet werden. In der Patentschrift DE 3 717 915 C2 wird ein solcher Retraktor beschrieben, der ohne eine gesonderte Hub-Vorrichtung funktioniert. Eine in einem festen Winkel zur Zahnstange des Retraktors stehende Valve verklemmt sich in der Hälfte des Sternums, welche nicht angehoben wird, sodass sich der Retraktor beim Spreizen mittels des Zahntriebes schräg zur normalen Brustfläche des Patienten aufstellt und die andere Sternumshälfte mit speziellen Valven, die als Krallen ausgebildet sind, nach oben zieht.

Eine Technik, die sich weitestgehend komplett von den drei oben beschriebenen Varianten unterscheidet, ist das Anheben einer Sternumshälfte mittels Seilen oder Zugstangen in Verbindung mit einer externen Befestigung. Diese Technik findet sich in den Druckschriften US 6,488,621 B1 oder US 6,689,053 B1. Hier wird nur die gewünschte Sternumshälfte mittels Haken, welche sich am einem Seil oder einer Zugstange befinden, angehoben, wobei die Zugkraft von einem Befestigungselement wie einem Rahmen oder einer Stange aufgenommen wird, das beispielsweise seitlich : am Operationstisch (OP-Tisch) befestigt ist.

Bei allen üblichen Vorrichtungen dieser Art muss sich der Anwender, das heißt der Operateur, auf einige Nachteile einstellen, die einerseits einen optimalen Ablauf und andererseits ein ideales Ergebnis des operativen Eingriffs verhindern.

Der größte Nachteil hierbei sind die hohen Kräfte, die beim Anheben des Sternums und dem Aufbiegen der daran anschließenden Rippen entstehen, und die meist durch Abstützelemente auf den Körper des Patienten abgeleitet werden. Dabei wird die betreffende Sternumshälfte exponiert, das heißt nach außen aufgebogen, die dafür erforderliche Kraft wird jedoch an den anschließenden Rippen derselben Körperseite abgeleitet. Dies führt aufgrund der verschiedenen Hebelarme zu sehr großen Krafteinwirkungen auf den Patienten, Damit besteht eine große Gefahr von Ausbrüchen des Sternums, Frakturen der Rippen und Schädigung von Nervenbahnen.

Eine Ausnahme bildet die Technik der externen Befestigung, da hier die Kraft für die Exponierung der Sternumshälfte auf das Befestigungssystem und nicht auf den Patienten abgeleitet wird. Allerdings können bei dieser Technik keine ausreichenden Sichtverhältnisse generiert werden, da nur eine vertikale Anhebung einer Sternumshälfte erfolgt und keine ausreichende Öffnung in horizontaler Achse vorhanden ist. Durch die fehlenden horizontalen Kräfte kann hier auch keine normale Valve verwendet werden, sondern es sind krallenförmige Haken notwendig, die nur sehr punktuell am Sternum anliegen und erhebliche Frakturen verursachen können. Zudem bauen derartige Systeme sehr hoch über die Operations-Ebene auf und sind daher bei vielerlei Arbeitsabläufen störend für das OP-Personal.

Ein weiterer Kritikpunkt ist das sehr umständliche Umbauen der Hub-Systeme auf die andere Seite, für den Fall, dass zwei Brustwandarterien (links und rechts) nacheinander präpariert werden müssen, Bei denjenigen Vorrichtungen, die am Retraktor adaptiert werden, geschieht dies noch relativ einfach, allerdings wird hier üblicherweise ein Helfer dem Operateur assistieren müssen. Die Retraktoren mit speziellen Valven müssen hingegen komplett aus der Operationsöffnung entnommen und umgebaut werden, was sowohl eine Unterbrechung des Operationsablaufs, als auch einen relativ kritischen Moment darstellt, da der Operateur bei entnommenem Retraktor keine Zugriffsmöglichkeit auf das Herzen hat.

Bei den Systemen mit einer externen Befestigungsstange, die am OP-Tisch montiert ist, ist ein sinnvolles Umsetzen auf die andere Seite kaum praktikabel. Hintergrund hierbei ist, dass alle Bereiche, die in vertikaler Richtung unterhalb des Operationsbereiches liegen, als unsteril gelten. Eine Befestigung am OP-Tisch befindet sich somit im unsterilen Bereich und darf nicht mehr abgenommen und an anderer Stelle angebracht werden. Genauso sind Arbeitsschritte, die während der Operation in diesem Bereich durchgeführt werden müssen, beispielsweise das Lösen einer Klemmung, untersagt.

### Kurzbeschreibung der Erfindung

Die Aufgabe der vorliegenden Erfindung ist es, eine Retraktorhalterung zu schaffen, die ein Exponieren einer Sternumshälfte zusammen mit dem Herstellen einer ausreichenden horizontalen Sternumöffnung ermöglicht und dabei den Patienten möglichst wenig belastet. Eine weitere Aufgabe der vorliegenden Erfindung ist es, eine Retraktorhalterung zu schaffen, die mit nur einer Hand bedienbar ist. Noch eine weitere Aufgabe der vorliegenden Erfindung ist es, eine solche Retraktorhalterung zu schaffen, die entweder mit einer Hand von einer Seite des OP-Tischs auf die andere Seite in einfacher Weise umbaubar ist oder einen solchen Umbau vollständig erübrigt.

Die Aufgabe der vorliegenden Erfindung wird durch eine teleskopierbare Retraktorhalterung gemäß Anspruch 1 gelöst. Weitere vorteilhafte Fortbildungen sind Gegenstand der abhängigen Ansprüche.

Gemäß einem Aspekt der vorliegenden Erfindung wird eine teleskopierbare Retraktorhalterung geschaffen mit einer teleskopierbaren Stütze, vorzugsweise einer teleskopierbaren Stützstange, weiter vorzugsweise mit einem Innenrohr, einem Außenrohr und einem Verriegelungsmechanismus zur Feststellung einer frei/individuell gewählten Stützstangenlänge sowie mit einem Retraktorhebel, vorzugsweise einer Retraktorstange, die an ihrem distalen Ende eine Retraktoraufnahme aufweist, welche daran angepasst ist, einen Retraktor lösbar zu greifen oder zu adaptieren, und welche an ihrem proximalen Ende über ein lösbares Verbindungsmittel an der teleskopierbaren Stützstange vorzugsweise lose gehalten oder abgestützt ist. Die teleskopierbare Stützstange ist dabei an einem Operationstisch abstützbar und weist einen Betätigungsmechanismus auf, durch den der Verriegelungsmechanismus von einer ersten Stellung, in welcher die Stützstangenlänge fixiert ist (das Innenrohr und das Außenrohr der teleskopierbaren Stützstange zueinander unverschieblich gehalten sind), in eine zweite Stellung überführbar ist, in welcher die Stützstangenlänge verstellbar ist (das Innenrohr und das Außenrohr der teleskopierbaren Stange relativ zueinander in Längsrichtung der Rohre bewegbar sind). Bei dieser Ausführungsform wird die erfindungsgemäße Retraktorhalterung bei der Verwendung am Patienten dadurch in einer im Wesentlichen aufrechten Position gehalten, indem der Retraktor am Sternum des Patienten befestigt ist und die Retraktoraufnahme den Retraktor greift. Auf diese Weise ergibt sich aus den Auflagerkräften des Sternums, der Abstützung der teleskopierbaren Stützstange und den Längen der teleskopierbaren Stützstange sowie der Retraktorstange eine eindeutige Lage der Retraktorhalterung. Die im Wesentlichen aufrechte Position der Retraktorhalterung bezieht sich auf die dann im Wesentlichen senkrechte Lage der teleskopierbaren Stange. In anderen Worten ausgedrückt, wird das (längs aufgesägte) Sternum mittels des Retraktors funktionsüblich (nach Art eines bekannten Mammaria-Retraktors) gespreizt bzw. aufgestemmt, wobei anschließend der Retraktor über die Retraktorstange verhebelt bzw. verschwenkt wird, in dem die Stützstange längenverstellt (ausgefahren) wird. Dadurch wird der Retraktor einseitig (auf Seiten der Stützstange) zusammen mit der entsprechenden Sternumshälfte angehoben bis für den Operateur optimale Sichtverhältnisse zu der Arteria Mammaria Interna vorliegen.

Die Länge der teleskopierbaren Stützstange lässt sich vorzugsweise verändern, indem mit Hilfe des Betätigungsmechanismus der Verriegelungsmechanismus gelöst wird, die Länge der teleskopierbaren Stützstange durch Anheben oder Absenken des Betätigungsmechanismus eingestellt wird, und anschließend der Betätigungsmechanismus freigegeben wird, sodass der Verriegelungsmechanismus die eingestellte Länge (Lage des Innenrohres und des Außenrohres der teleskopierbaren Stützstange zueinander) verriegelt. Im einfachsten Fall, der hier zunächst dargestellt ist, stützt sich die teleskopierbare Stützstange auf der Oberfläche eines Operationstisches ab. Um ein Wegrutschen der teleskopierbaren Stange zu verhindern, können Vertiefungen in dem Operationstisch vorgesehen sein.

Alternativ oder zusätzlich zur teleskopierbaren Retraktorstange ist es aber auch möglich, das lösbare Verbindungsmittel zwischen Retraktrostange und Stützstange so zu gestalten, dass die wirksame Retraktorstangenlänge zwischen Stützstange und Retraktoraufnahme veränderbar ist. Beispielsweise kann die Retraktorstange im Bereich des Verbindungsmittels eine Anzahl von längsbeabstandeten An-/Eingriffsstellen aufweisen, an denen die Retraktorstange lösbar mit der Stützstange in Eingriff/Auflage bringbar ist. Auch kann das Verbindungsmittel so gestaltet sein, um eine Längsverschiebung der daran gehaltenen Retraktorstange zuzulassen.

Gemäß einem weiteren Aspekt der vorliegenden Erfindung ist der Betätigungsmechanismus im Bereich des Verbindungsmittels mit der Retraktorstange angeordnet. Das Verbindungsmittel liegt im Wesentlichen stets in der Ebene, die der Retraktor aufspannt. Der Betätigungsmechanismus liegt daher ebenfalls in diesem Bereich und somit stets im sterilen Bereich. Der Operateur kann somit die Retraktorhalterung bedienen, ohne dass eine erneute Sterilisation des Arztes erfolgen muss.

Gemäß einem anderen, ggf. unabhängig zu beanspruchenden Aspekt der vorliegenden Erfindung ist das Innenrohr der teleskopierbaren Stützstange an einem Operationstisch abstützbar, das Außenrohr der teleskopierbaren Stützstange ist an einem Ende über das lösbare Verbindungsmittel mit der Retraktorstange wirkverbunden und der Betätigungsmechanismus ist im Bereich des anderen Endes des Außenrohrs angeordnet. In diesem Fall ist der Betätigungsmechanismus etwas tiefer angeordnet, als sich dies im obigen Fall darstellt, er befindet sich aber dennoch in einem Bereich, der als steril angesehen werden kann.

Gemäß einem weiteren Aspekt der vorliegenden Erfindung ist die Retraktoraufnahme daran angepasst, einen Retraktor formschlüssig zu greifen bzw. zu adaptieren. Auf diese Weise können die Kräfte und Momente des Retraktors gut auf die Retraktorhalterung und umgekehrt übertragen werden.

Gemäß einem weiteren Aspekt der vorliegenden Erfindung ist die Retraktoraufnahme eine im Wesentlichen U-förmige Aussparung, in die ein Rahmen eines Retraktors einführbar ist, sodass dieser an wenigstens zwei Seiten zumindest teilweise in Anlage an die Innenwand der Aussparung gelangt. Eine elastisch vorgespannte Zunge als Rastmittel weist an ihrem distalen Ende einen Verriegelungsvorsprung auf, der daran angepasst ist, bei einem eingeführten Retraktorrahmen an diesem anzuliegen und dadurch einen Formschluss mit dem Retraktorrahmen herzustellen. Im einfachsten Fall liegt die U- förmige Aussparung an drei Seiten eines Retraktorbauteils/Retraktorrahmens an und der Verriegelungsvorsprung liegt an der vierten Seite des Retraktorbauteils/Retraktorrahmens an, um ein Herausrutschen desselben aus der Retraktoraufnahme zu verhindern. Das gegriffene Retraktorbauteil muss dabei nicht rechtwinklig sein. Vorteilhaft ist lediglich, wenn das gegriffene Retraktorbauteil nicht rotationssymmetrisch ist. In diesem Fall müsste eine kraftschlüssige Verbindung zwischen Retraktor und Retraktoraufnahme hergestellt werden, was einfach möglich ist aber die Bedienung durch den Operateur während einer Operation verkompliziert. Gemäß einem weiteren Aspekt der vorliegenden Erfindung ist die Zunge drehbar an einer Seite der Aussparung angebracht und ist durch ein elastisches Bauteil so vorgespannt, dass ihr distales Ende mit dem Verriegelungsvorsprung zu der Aussparung hin drängt. Dies entspricht einer vorteilhaften Anordnung, da der Operateur die Retraktoraufnahme vom Retraktor lösen kann, indem er mit einer Hand die Retraktorstange greift und beispielsweise mit dem Daumen derselben Hand die Zunge an ihrem proximalen Ende drückt, um die Hinterschneidung des Verriegelungsvorsprungs an dem distalen Ende der Zunge mit der abgewandten Seite des Retraktorbauteils aufzuheben, um den Retraktor von der Retraktoraufnahme zu lösen. Denkbar sind aber auch zwei gegenüberliegende Zungen, die sich spiegelsymmetrisch zueinander verhalten. Auch denkbar ist eine Zunge, die in ein Retraktorbauteil eingeführt wird und deren distales Ende von der U-förmigen Aussparung weg vorgespannt ist.

Gemäß einem weiteren Aspekt der vorliegenden Erfindung ist das elastische Bauteil eine Druckfeder, die das proximale Ende der Zunge drückt. In diesem Fall kann das elastische Bauteil im Bereich des Bodens der U-förmigen Aussparung angeordnet sein. Diese Anordnung eignet sich auch für eine Retraktoraufnahme mit zwei Zungen, wobei je eine Druckfeder pro Zunge oder aber eine gemeinsame Druckfeder vorgesehen sein kann.

Gemäß einem weiteren Aspekt der vorliegenden Erfindung weist die Zunge an dem proximalen Ende einen Betätigungsvorsprung zum Freigeben eines Retraktors aus der Retraktoraufnahme auf. Dies vereinfacht es dem Operateur, die Zunge zu ertasten, und es erleichtert ihm auch das Drücken des proximalen Endes der Zunge. Die Zunge kann so auch kleiner ausgebildet werden, da die Vertiefung zur Aufnahme des proximalen Endes der Zunge nicht zur Aufnahme des Daumens oder eines anderen Betätigungsfingers des Operateurs ausgestaltet werden muss.

Gemäß einem weiteren Aspekt der vorliegenden Erfindung weist das lösbare Verbindungsmittel zumindest eine, vorzugsweise mehrere längsbeabstandete Hinterschneidungen/Einkerbungen/Eindrehungen/etc. an der Retraktorstange auf, die mit einer Haltekante oder Quer-/Schwenkbolzen am freien Ende der Stützstange (durch einfache Auflage) ausgewählt zusammenwirken und somit ein unbeabsichtigtes Längsverschieben des Reaktorstange an der Stützstange verhindern. Es besteht auch die Möglichkeit, das Verbindungsmittel als eine Klemme (Rohrschelle, etc.) vorzusehen, die schwenkbar und /oder rotierbar an der Abstützstange gehalten ist und die Retraktorstange wahlweise reibschlüssig hält.

Gemäß einem weiteren Aspekt der vorliegenden Erfindung ist der Betätigungsmechanismus ein Ring ist, welcher relativ zu der teleskopierbaren Stange bewegbar ist, um den Verriegelungsmechanismus von der ersten Stellung in die zweite Stellung zu überführen. Vorteilhaft ist hierbei ein Ring, der gegenüber dem Rohr, an dem er vorgesehen ist, in Längsrichtung dieses Rohres verschiebbar ist, um so den Verriegelungsmechanismus frei zu geben. Der Betätigungsmechanismus kann aber auch in verschiedenen weiteren Formen ausgebildet sein, so zum Beispiel als Betätigungsknopf oder Hebel, der gedrückt wird, um den Verriegelungsmechanismus frei zu geben, oder als Ring oder ein anderes Bauteil, welches zur Freigabe des Verriegelungsmechanismus relativ zu dem betreffenden Rohr verdreht wird, sei es um die Achse des Rohres oder in einer anderen Richtung. Auch kann die Stützstange als Spindelstange oder Zahnstange ausgebildet sein, wobei im Fall einer Spindel der Betätigungsmechanismus eine Stellschraube ist, bei deren Drehen die Spindel (inneres Rohr) längs des Spindelzylinder (äußeres Rohr) verschoben wird. Sollte eine Zahnstange vorgesehen sein, wäre ein Kurbelantrieb als Betätigungsmechanismus denkbar.

Gemäß einem weiteren Aspekt der vorliegenden Erfindung weist die teleskopierbare Stützstange an ihrem dem lösbaren Verbindungsmittel gegenüberliegenden Endabschnitt eine im Wesentlichen U-förmige Befestigungsaufnahme auf, um von oben auf eine Operationstischschiene aufsetzbar zu sein. Im einfachsten Fall wird diese Befestigungsaufnahme einfach über eine Operationstischschiene gesetzt, ohne dass irgendeine Art von Formschluss oder enger Anlage entsteht. Auf diese Weise kann sich die teleskopierbare Stange in alle Richtungen hinreichend gegenüber der Operationstischschiene verdrehen und sich so den Kraft- und Momentenverhältnissen des Systems anpassen.

Gemäß einem weiteren Aspekt der vorliegenden Erfindung ist der Querschnitt der Befestigungsaufnahme an einen Querschnitt einer Operationstischschiene angepasst, um im aufgesetzten Zustand der Befestigungsaufnahme zumindest teilweise an beiden Seitenwänden der Operationstischschiene in Anlage zu kommen. Hierbei ist die Befestigungsaufnahme über eine Gelenkverbindung an der teleskopierbaren Stange angebracht. Die Verdrehung der teleskopierbaren Stange gegenüber der Operationstischschiene dient dazu, dass keine zu großen Kräfte vom Sternum auf den Retraktor und anschließend auf die Retraktorhalterung und den Operationstisch sowie in umgekehrter Richtung übertragen werden, um eine Verletzung des Sternums oder des umgebenden Gewebes des Patienten sicher zu vermeiden. Die wichtigste Verdrehung der teleskopierbaren Stange gegenüber dem Operationstisch ist hierbei eine Verdrehung um die Längsachse des Operationstisches. Die Verdrehungen in andere Richtungen entstehen überwiegend durch Fehllagerungen des Patienten auf dem Operationstisch, durch nicht gerade ausgeführte Schnitte durch das Sternum und durch das weitere Aufspreizen des Sternums. Diese Verdrehungen sind aber von einer Größenordnung, die mittels Spiel in den verschiedenen Abstützungssystemen aufgenommen werden kann. Zudem besteht für den Operateur stets die Möglichkeit, die Lage des Abstützungspunkts der teleskopierbare Stange entlang der Körperlängsachse des Patienten so anzupassen, dass die angesprochenen unbeabsichtigten Verdrehungen minimiert werden.

Gemäß einem weiteren Aspekt der vorliegenden Erfindung ist die Gelenkverbindung zwischen der teleskopierbaren Stange und der Befestigungsaufnahme so ausgebildet, dass eine Drehbewegung zwischen der teleskopierbaren Stange und der Befestigungsaufnahme nur in einem vorbestimmten Winkelbereich möglich ist. Dieser. Winkelbereich ist hinreichend groß, damit die Längenveränderungen der teleskopierbaren Stange von maximaler Länge zu minimaler Länge oder anders herum durchgeführt werden können, ohne dass eine Winkelbegrenzung stattfindet. Diese Winkelbegrenzung dient vielmehr dazu, zu verhindern, dass die Retraktoraufnahme umkippt und in den unsterilen Bereich eintritt, wenn der Operateur die Befestigungsaufnahme bereits direkt oder indirekt an der Operationstischschiene befestigt hat, aber die Retraktoraufnahme noch nicht an dem Retraktor befestigt hat, In diesem Zustand kann die Retraktorhalterung ohne eine Winkelbegrenzung von dem Patienten weg kippen und somit in den unsterilen Bereich kippen. Mit einer Winkelbegrenzung kann sie nur um einen vorbestimmten Winkel wegkippen und der Bereich der Retraktorhalterung, an dem der Operateur die Retraktorhalterung greift und betätigt, bleibt weiterhin im sterilen Bereich. Dies macht den Einsatz einer solchen Retraktorhalterung noch sicherer.

Gemäß noch einem weiteren Aspekt der vorliegenden Erfindung weist die teleskopierbare Retraktorhalterung mindestens einen Befestigungsmechanismus auf, welcher eine Befestigungsschiene aufweist und daran angepasst ist, lösbar an einer Operationstischschiene befestigbar zu sein. Dabei weist die teleskopierbare Stützstange an ihrem dem lösbaren Verbindungsmittel gegenüberliegenden Endabschnitt eine Befestigungsaufnahme auf, die auf die Befestigungsschiene aufsetzbar bzw. aufschiebbar ist. Wenn die Retraktorhalterung an einer Operationstischschiene abgestützt bzw. an dieser befestigt werden soll, besteht nämlich das Problem, dass diese Operationstischschienen nicht durchgängig entlang der gesamten Länge des Operationstischs vorgesehen sind. Dies hat unter anderem damit zu tun, dass Operationstische segmentiert sind, um verschiedenste Einstellungen an den Patienten und die Operation zu ermöglichen. Des Weiteren sind die Operationstischschienen in der Regel von sterilen Tüchern abgedeckt und damit nicht einsehbar. Wird daher die Retraktorhalterung längs der Operationstischschiene verschoben, könnten daran liegende Kabel/Leitungen/etc. unabsichtlich beschädigt werden. Um diese Probleme zu lösen, verfügt die erfindungsgemäße teleskopierbare Retraktorhalterung über den vorstehend genannten als eine separate Baueinheit ausgebildeten Befestigungsmechanismus, der seinerseits an der Operationstischschiene befestigbar ist; und bietet eine eigene Befestigungsschiene (oberhalb der Operationstücher und damit für den Operateur sichtbar), an der dann die teleskopierbare Stützstange der teleskopierbaren Retraktorhalterung abstützbar ist. Der Begriff Aufschieben bezeichnet hier ein Aufschieben der Befestigungsaufnahme auf die Befestigungsschiene in deren Längsrichtung.

Gemäß einem weiteren Aspekt der vorliegenden Erfindung weist die Befestigungsschiene des separaten Befestigungsmechanismus einen Querschnitt auf, der aus einer im Wesentlichen kreisförmigen Fläche und einer weiteren Fläche zusammengesetzt ist. Die Befestigungsaufnahme der teleskopierbaren Stützstange weist eine Aussparung mit einer Innenwand und zwei Seitenwänden auf, wobei der Querschnitt der Aussparung im Wesentlichen dem Querschnitt der Befestigungsschiene öntspricht. Die Innenwand der Aussparung stellt mit der Befestigungsschiene im aufgeschobenen Zustand mindestens eine Hinterschneidung her, die in der Lage ist, Zugkräfte von der teleskopierbaren Stange auf die Befestigungsschiene zu übertragen. Zudem sind die Seitenwände der Aussparung so zu den Seitenflächen der weiteren Fläche der Querschnittsfläche der Befestigungsschiene beabstandet und ausgerichtet, dass eine Verdrehung der teleskopierbaren Stange relativ zu der Befestigungsschiene auf einen vorbestimmten Winkelbereich begrenzt ist. Der Querschnitt der Befestigungsschiene bestehend aus rotationssymmetrischem Bestandteil und weiterem nicht rotationssymmetrischem Bestandteil, also zumindest nicht zum selben Zentrum rotationssymmetrisch wie der erste Bestandteil, sowie die zugehörige Befestigungsaufnahme dienen dazu, eine Verdrehung der teleskopierbaren Stange gegenüber der Befestigungsschiene in einem gewissen Winkelbereich zu ermöglichen und gleichzeitig eine weitergehende Verdrehung zu verhindern. Die einfachste zusammengesetzte Form dieser Art ist die Form eines Schlüssellochs bestehend aus einer Kreisfläche und einer sich anschließenden Kreissegmentfläche, die nicht voll rotationssymmetrisch ist.

Gemäß einem weiteren Aspekt der vorliegenden Erfindung ist die Befestigungsaufnahme an einem Ende der teleskopierbaren Stützstange angeordnet. Auf diese Weise kann das untere Rohr der teleskopierbaren Stützstange und die Befestigungsaufnahme aus einem Stück ausgebildet werden.

Gemäß einem weiteren Aspekt der vorliegenden Erfindung ist die Retraktorstange mit dem Innenrohr der teleskopierbaren Stange lösbar/lose wirkverbunden, der Betätigungsmechanismus ist vorzugsweise an dem Innenrohr der teleskopierbaren Stützstange vorgesehen und die Befestigungsaufnahme ist vorzugsweise an einer Seitenwand des Außenrohrs der teleskopierbaren Stützstange vorgesehen. Mit einer seitlich am Außenrohr der teleskopierbaren Stützstange vorgesehenen Befestigungsaufnahme kann die Länge der teleskopierbaren Stützstange vergrößert werden, ohne dass sich die Höhe des Systems über dem Operationstisch vergrößert, was zu einer Störung des Operateurs führen würde, beispielsweise zu einer Einschränkung seiner Sicht oder seiner Bewegungsfreiheit. Die größere Länge der teleskopierbaren Stützstange ist insbesondere dann von Vorteil, wenn mit Hilfe der teleskopierbaren Retraktorhalterung nicht nur die Seite des Sternums, auf der sich die Retraktorhalterung befindet, angehoben werden soll, indem die Länge der teleskopierbaren Stützstange vergrößert wird, sondern auch die andere Sternumshälfte angehoben werden soll, indem die Länge der teleskopierbaren Stützstange verringert wird. Hierbei stützt sich der Retraktor jeweils auf der Sternumshälfte auf, die nicht angehoben wird, was leicht möglich ist, da Rippen sich wesentlich leichter aufbiegen als eindrücken lassen. Damit aber eine ausreichende Länge der teleskopierbaren Stützstange erreicht wird, ohne dass die Höhe des Systems über dem Operationstisch so groß wird, dass der Operateur beeinträchtigt wird, ist es vorteilhaft, die teleskopierbare Stützstange unter die Operationstischschiene zu verlängern. Um dann aber die teleskopierbare Stützstange noch an der Operationstischschiene befestigen zu können, muss die Befestigungsaufnahme seitlich an der teleskopierbaren Stützstange angebracht sein.

Gemäß einem weiteren Aspekt der vorliegenden Erfindung weist die teleskopierbare Stützstange mindestens ein weiteres Mittelrohr auf, wobei das mindestens eine weitere Mittelrohr zwischen dem Innenrohr und dem Außenrohr angeordnet ist und mit diesen zusammen eine mehrfach teleskopierbare Stange bildet. Auf diese Weise kann die teleskopierbare Länge vergrößert werden, ohne dass die Länge der teleskopierbaren Stützstange im zusammengeschobenen Zustand vergrößert wird.

### Figurenbeschreibung

Weitere Vorteile und Merkmale der Retraktorhalterung sind dem Fachmann aus den beigefügten Figuren und der detaillierten Beschreibung der Ausführungsbeispiele ersichtlich.
Fig. 1 zeigt ein erstes Ausführungsbeispiel der teleskopierbaren Retraktoraufnahme zum Aufstützen auf einem Operationstisch;
Fig. 2 zeigt ein zweites Ausführungsbeispiel der teleskopierbaren Retraktoraufnahme zum Aufstützen auf einem Operationstisch;
Fig. 3 zeigt ein drittes Ausführungsbeispiel der teleskopierbaren Retraktoraufnahme zum Aufstützen auf einem Operationstisch;
Fig. 4 zeigt ein viertes Ausführungsbeispiel der teleskopierbaren Retraktoraufnahme zum Abstützen an einer Operationstischschiene;
Fig. 5 zeigt ein fünftes Ausführungsbeispiel der teleskopierbaren Retraktoraufnahme zum Abstützen an einer Operationstischschiene;
Fig. 6 zeigt ein sechstes Ausführungsbeispiel der teleskopierbaren Retraktoraufnahme mit Befestigungsmechanismus;
Fig. 7 zeigt ein siebtes Ausführungsbeispiel der teleskopierbaren Retraktoraufnahme mit Befestigungsmechanismus;
Fig. 8 zeigt eine seitliche Ansicht einer Retraktoraufnahme;
Fig. 9 zeigt eine seitliche Schnittansicht gemäß Fig. 8;
Fig. 10 zeigt einen Befestigungsmechanismus und eine Befestigungsaufnahme des sechsten Ausführungsbeispiels im Detail;
Fig. 11 zeigt schematisch einen Operationstisch sowie den Brustkorb eines Patienten sowie eine teleskopierbare Retraktoraufnahme entsprechend dem sechsten Ausführungsbeispiel:
Fig. 12 zeigt eine Ansicht eines Patienten auf einem Operationstisch von oben;
Fig. 13 zeigt eine Ansicht eines Patienten auf einem Operationstisch von der Seite;
Fig. 14 zeigt ein achtes, erfindungsgemäßes Ausführungsbeispiel der teleskopierbaren Retraktoraufnahme mit Befestigungsmechanismus in einer Perspektivendarstellung;
Fig. 15 zeigt das achte Ausführungsbeispiel der teleskopierbaren Retraktoraufnahme mit Befestigungsmechanismus in einer Seitendarstellung;
Fig. 16 zeigt in der Perspektivenansicht eine Variante eines lösbaren Verbindungsmittels zwischen der teleskopierbaren Stützstange und der Retraktorstange gemäß der vorliegenden Erfindung in gelöstem Zustand;
Fig. 17 zeigt in der Perspektivenansicht die Variante eines lösbaren Verbindungsmittels zwischen der teleskopierbaren Stützstange und der Retraktorstange gemäß Fig. 16 in wirkverbundenem Zustand;
Fig. 18 zeigt in der Seitenansicht die Variante eines lösbaren Verbindungsmittels zwischen der teleskopierbaren Stützstange und der Retraktorstange gemäß Fig. 16 in wirkverbundenem Zustand; und
Fig. 19 zeigt eine weitere Variante einer Stützstange mit Befestigungsaufnahme nach dem Prinzip einer Spindel.

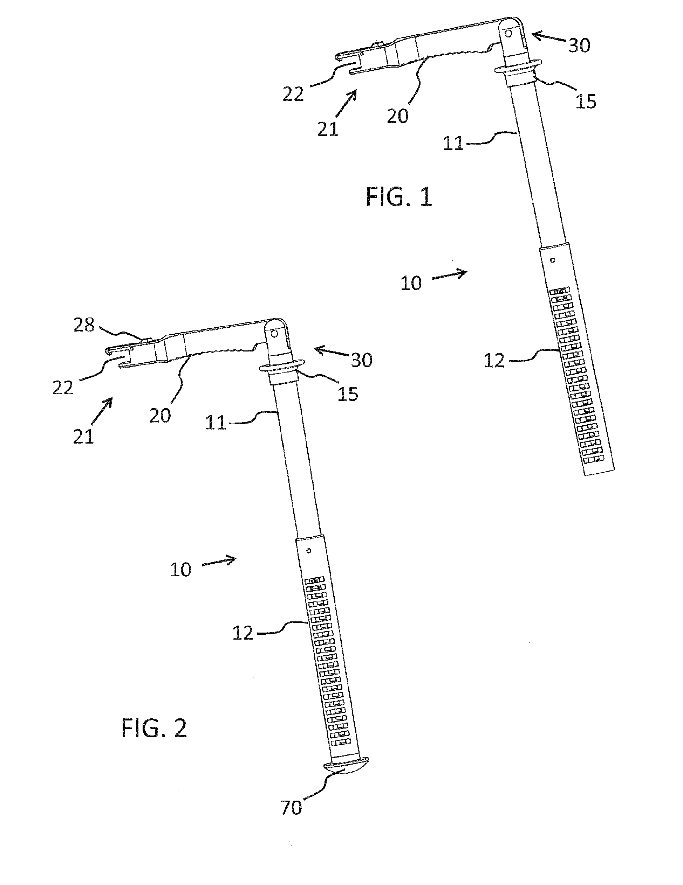

Ein erstes Ausführungsbeispiel ist unter Bezugnahme auf die Fig. 1 im Detail beschrieben.

Die teleskopierbare Retraktorhalterung gemäß dem ersten Ausführungsbeispiel hat eine teleskopierbare Stützstange 10 vorzugsweise mit einem Innenrohr 11, einem Außenrohr 12 und einem Verriegelungsmechanismus sowie eine Retraktorstange 20. Die Retraktorstange 20 weist an ihrem einen, distalen Ende eine Retraktoraufnahme 21 auf, die einen Retraktor R lösbar greifen kann. An ihrem anderen, proximalen Ende ist die Retraktorstange 20 über ein lösbares/loses Verbindungsmittel 30 mit der teleskopierbaren Stützstange 10 wirkverbunden. Die teleskopierbare Stützstange 10 ist gemäß diesem Ausführungsbeispiel an einem Operationstisch 0 abstützbar, indem sie mit dem freien (dem Verbindungsmittel gegenüberliegenden) Ende des Außenrohrs 12 einfach auf den Operationstisch 0 gestellt wird oder in spezielle Vertiefungen des Operationstischs 0 gesetzt wird. Die teleskopierbare Stützstange 10 weist an ihrem Innenrohr 11 einen Betätigungsmechanismus 15 auf, durch den der Verriegelungsmechanismus von einer ersten Stellung, in welcher das Innenrohr 11 und das Außenrohr 12 der teleskopierbaren Stützstange 10 zueinander unverschieblich gehalten sind, in eine zweite Stellung überführbar ist, in welcher das Innenrohr 11 und das Außenrohr 12 der teleskopierbaren Stützstange 10 relativ zueinander in Längsrichtung der Rohre 11,12 bewegbar sind. Zwischen dem Innenrohr 11 und dem Außenrohr 12 besteht ein gewisses Spiel derart, dass sich die beiden Rohre 11,12 in einem bestimmten Bereich zueinander um ihre Längsachsen verdrehen können.

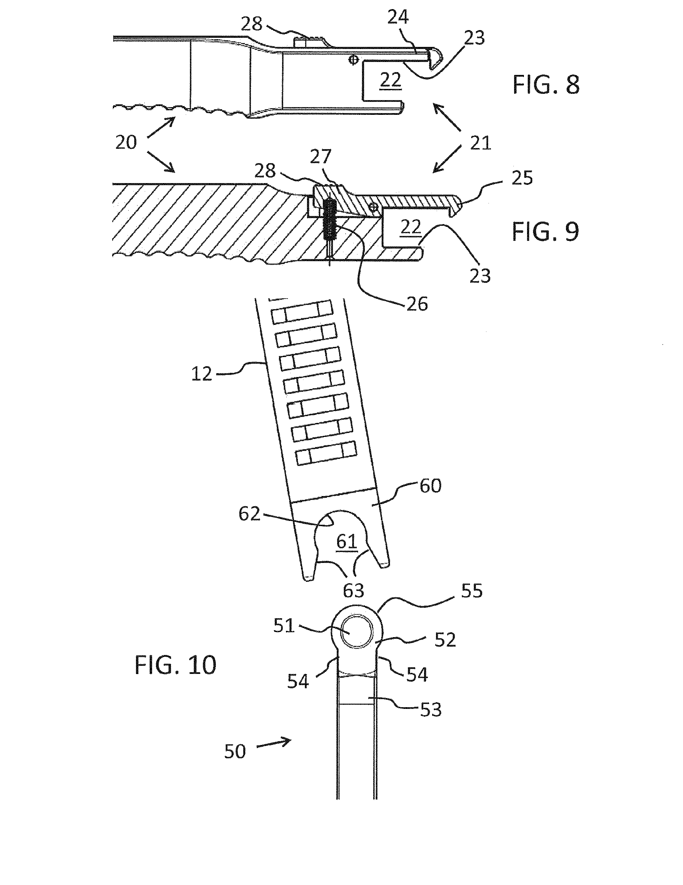

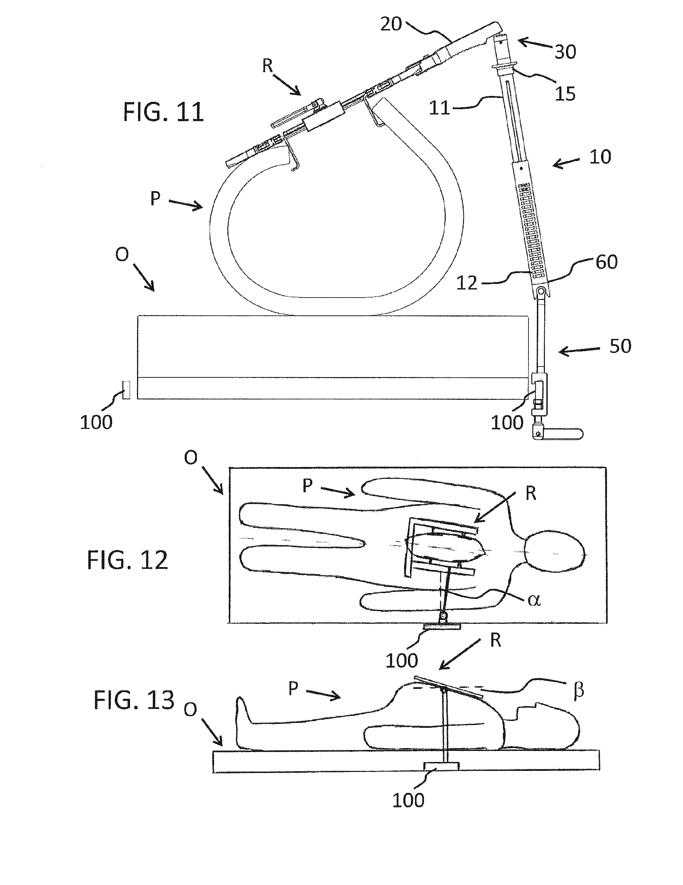

Durch das Aufsetzen der teleskopierbaren Stützstange 10 auf dem Operationstisch 0 ist die teleskopierbare Stützstange 10 gelenkig an dem Operationstisch 0 abstützbar. Der Betätigungsmechanismus 15 ist direkt unterhalb der Gelenkverbindung 30 mit der Retraktorstange 20 an dem Innenrohr 11 angeordnet. Die Retraktoraufnahme 21 ist bei diesem Ausführungsbeispiel daran angepasst, einen Retraktor R formschlüssig zu greifen. Dafür hat die Retraktoraufnahme 21, wie dies in den Fig. 8 und 9 gezeigt ist, eine im Wesentlichen U-förmige Aussparung 22, in die ein Rahmen eines Retraktors R einführbar ist. In der Fig. 11 ist gezeigt, dass der Retraktor R an drei Seiten an der Innenwand 23 der Aussparung 22 anliegt und eine elastisch vorgespannte Zunge 24 an ihrem distalen Ende einen Verriegelungsvorsprung 25 aufweist, der den Retraktor R in der U-förmigen Aussparung formschlüssig hält. Die Zunge 24 ist drehbar an einer Seite der Aussparung 22 angebracht und durch eine Druckfeder 26 so vorgespannt, dass ihr distales Ende mit dem Verriegelungsvorsprung 25 zu der Aussparung 22 hin gedrückt wird. Außerdem weist die Zunge 24 an ihrem proximalen Ende 27 einen Betätigungsvorsprung 28 auf, der durch den Operateur betätigt bzw. gedrückt werden kann, um den Retraktor R aus der Retraktoraufnahme frei zu geben.

Bei diesem Ausführungsbeispiel bildet zudem ein Scharniergelenk vorzugsweise mit abziehbarem Splintbolzen als Schnellverschluss das lösbare Verbindungsmittel 30 zwischen Innenrohr 11 der teleskopierbaren Stützstange 10 und der Retraktorstange 20. Der Betätigungsmechanismus 15 ist hier ein Ring, der relativ zu der teleskopierbaren Stützstange 10 in Richtung des Scharniergelenks 30 bewegbar ist, um den Verriegelungsmechanismus von der ersten Stellung in die zweite Stellung zu überführen.

Die teleskopierbare Retraktorhalterung gemäß diesem Ausführungsbeispiel wird folgendermaßen verwendet. Nachdem der Operateur das Sternum des Patienten P geöffnet und den Retraktor R eingesetzt und gespreizt hat, greift er die teleskopierbare Retraktoraufnahme im Bereich des lösbaren Verbindungsmittels 30 und setzt die teleskopierbare Stützstange 10 auf dem Operationstisch 0 auf. Danach legt er seinen Daumen auf das lösbare Verbindungsmittel (Scharniergelenk) 30 und greift mit Zeigefinger und Ringfinger den Ring 15 und zieht ihn in Richtung seines Daumens, um so die Verriegelung der teleskopierbaren Stützstange 10 aufzuheben. Jetzt passt er die Länge der teleskopierbaren Stützstange 10 an die Bedingungen des Patienten P an, das bedeutet im Wesentlichen an die Größe des Brustkorbs des Patienten P. Während er mit der einen Hand nach wie vor die Retraktorhalterung im Bereich des lösbaren Verbindungsmittels 30 hält, führt er mit der anderen Hand die Retraktoraufnahme 21 in die Richtung des Retraktors R bzw. der vorgesehenen Befestigungsstelle an dem Retraktors R. Dies ist üblicherweise einer der beiden Schenkel eines Retraktors, die parallel zu dem Sternumsschnitt verlaufen und die Valven tragen. Jetzt führt er den Retraktor R in die Retraktoraufnahme 21 ein, indem er die Retraktoraufnahme 21 über den Retraktor R schiebt. Nun kann der Operateur die Seite des Sternums des Patienten P anheben, auf deren Seite die Retraktorhalterung positioniert ist, indem er erneut den Betätigungsmechanismus betätigt und das lösbare Verbindungsmittel 30 anhebt. Da die Retraktorstange 20 lösbar aber fest mit dem Retraktor R verbunden ist und somit eine Art Kragarm des Retraktors R bildet, ergibt sich ein günstiger Hebel für das Anheben bzw. Exponieren der einen, der Stützstange 10 zugewandten Sternumshälfte. Auf diese Weise benötigt der Operateur weniger Kraft zur Sternumsexposition. Hinzu kommt noch die Tatsache, dass die Rippen leichter (d.h. mit deutlich weniger Kraft) nach außen gebogen werden können, als sie in den Brustraum hinein gebogen werden können. Beim Anheben der Retraktorstange 20 werden somit die Rippen auf der Seite des Retraktors, auf der die teleskopierbare Retraktorhalterung angebracht ist, nach außen gebogen. Dies verursacht eine Kraft, die die Rippen auf der anderen Seite des Brustkorbs tendenziell nach innen drückt. Gegen solch eine Verformung bieten die Rippen allerdings einen großen Widerstand, sodass sie quasi als Widerlager für die Exposition der gegenüberliegenden Sternumshälfte dienen.

Lässt der Operateur nun die Retraktorhalterung los, stützt sich die Retraktorhalterung auf dem Operationstisch 0 auf und hält die eine Sternumshälfte hoch. Die zuvor beschriebenen Verdrehungen α und ß aufgrund der Fehllänge des Patienten P und der caudal und cranial unterschiedlichen Eigenschaften der Rippen werden durch eine Verdrehung und ein leichtes Kippen der Retraktoraufnahme ausgeglichen.

Möchte der Operateur im Laufe der Operation die andere Seite des Sternums des Patienten P anheben, bringt er den Retraktor R zunächst in eine annähernd horizontale Lage, indem er die Länge der teleskopierbaren Stützstange 10 angemessen verringert, löst die Retraktoraufnahme 21 von Retraktors R, setzt die Retraktorhalterung auf die andere Seite des Patienten P, befestigt die Retraktoraufnahme 21 an dem anderen Schenkel des Retraktors R und verlängert die teleskopierbare Stützstange 10 wieder. All dies kann der Operateur tun, ohne den sterilen Bereich zu verlassen. Geschickte Operateure benötigen für den gesamten Vorgang auch nur eine einzige Hand und müssen ihre zweite Hand nicht einmal verwenden, um die Retraktoraufnahme an dem Retraktor R zu befestigen oder den Retraktor R von der Retraktoraufnahme zu lösen.

Im Folgenden ist ein zweites Ausführungsbeispiel unter Bezugnahme auf die Fig. 2 beschrieben. Das zweite Ausführungsbeispiel unterscheidet sich von dem ersten Ausführungsbeispiel lediglich durch den Auflageteller 70 am freien Ende der teleskopierbaren Stützstange 10. Bei dem ersten Ausführungsbeispiel kann es sein, dass das freie Ende der teleskopierbaren Stützstange 10 wegrutscht, wenn die Reibung zwischen der teleskopierbaren Stützstange 10 und dem Operationstisch 0 nicht ausreichend groß ist oder jemand gegen die Retraktorhalterung stößt. Um die Auflage der Retraktorhalterung auf dem Operationstisch 0 zu verbessern, ist ein Auflageteller 70 vorgesehen, der vorzugsweise eine Kugelflächen- oder Kugelkalottenform an seiner Unterseite aufweist. Hierdurch wird ein Wegrutschen der Retraktorhalterung unwahrscheinlicher.

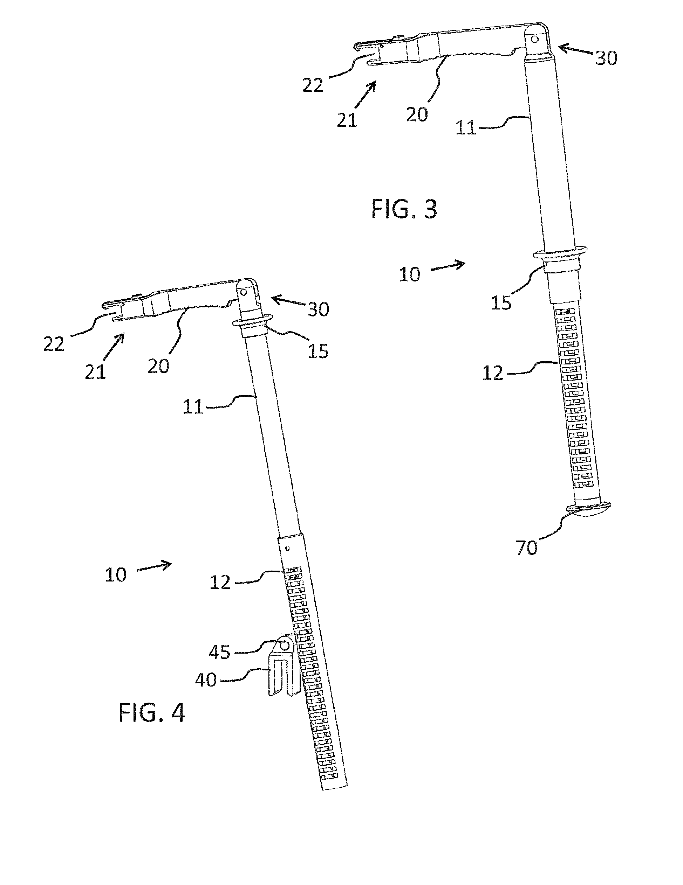

Im Folgenden ist ein drittes Ausführungsbeispiel unter Bezugnahme auf die Fig. 3 beschrieben. Das dritte Ausführungsbeispiel unterscheidet sich von dem zweiten Ausführungsbeispiel dadurch, dass bei dem zweiten Ausführungsbeispiel das Innenrohr 11 das obere Rohr bildet und das Außenrohr 12 das untere Rohr bildet, wohingegen beim dritten Ausführungsbeispiel das Innenrohr 11 das untere Rohr bildet und das Außenrohr 12 das obere Rohr bildet. Eine nicht gezeigte Alternative zu dem Auflageteller 70 des zweiten und dritten Ausführungsbeispiels ist ein ähnlicher Teller, der mit der flachen Seite auf dem Operationstisch 0 aufliegt und mittels eines lösbaren Kugelgelenks an dem freien Ende der teleskopierbaren Stützstange angebracht ist.

Im Folgenden ist ein viertes Ausführungsbeispiel unter Bezugnahme auf die Fig. 4 beschrieben. Das dritte Ausführungsbeispiel unterscheidet sich von dem zweiten Ausführungsbeispiel durch eine Befestigungsaufnahme 40, die in diesem besonderen Fall seitlich an dem Außenrohr 12 der teleskopierbaren Stützstange 10 angebracht ist. Die Befestigungsaufnahme 40 ist über ein Scharniergelenk 45 an der teleskopierbaren Stützstange 10 angebracht und weist einen U-förmigen Abschnitt auf, der passend auf eine Operationstischschiene aufgesetzt werden kann. Passend bedeutet hier, dass die Befestigungsaufnahme 40 an drei Seiten in Anlage an eine Operationstischschiene gelangt, wobei sie dennoch entlang der Operationstischschiene verschiebbar ist. Zudem ist die teleskopierbare Stützstange 10 länger als bei den vorangehenden Ausführungsbeispielen ausgebildet, sodass es sich über die Befestigungsaufnahme 40 und damit über die Operationstischschiene hinaus in Richtung Boden erstreckt. Der übrige Aufbau sowie die Funktionsweise entspricht dem ersten bis dritten Ausführungsbeispiel, wobei ein Kippen der Retraktorhalterung zu dem Patienten P hin durch das Scharniergelenk 45 ermöglicht wird, ein Kippen entlang der Längsachse des Operationstisches (Winkel ß) ermöglicht wird, indem sich die teleskopierbare Stützstange 10 gegenüber der Operationstischschiene leicht verdreht, und wobei sich eine Fehllage des Retraktors R in der Horizontalen (Winkel α) durch ein gleichzeitiges Verschieben und Kippen der Befestigungsaufnahme 40 ausgleichen lässt. Die Einhandbedienung sowie ein einhändiges Umsetzen von einer Seite des Patienten P auf die andere Seite funktioniert wie bei den vorangehenden Ausführungsbeispielen.

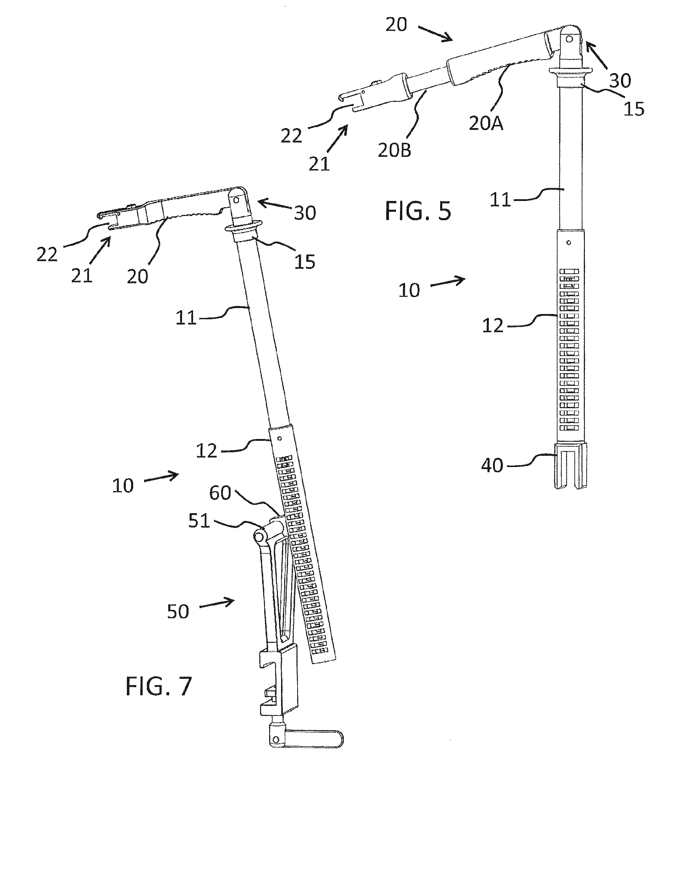

Im Folgenden ist ein fünftes Ausführungsbeispiel unter Bezugnahme auf die Fig. 5 beschrieben. Dieses besonders vorteilhafte Ausführungsbeispiel unterscheidet sich von dem vierten Ausführungsbeispiel dadurch, dass die Befestigungsaufnahme 40 nicht gelenkig sondern starr an der teleskopierbaren Stützstange 10 angebracht ist. Demzufolge kann sich die teleskopierbare Stützstange 10 nicht zu dem Patienten P hin neigen. Wenn bei einem der vorangehenden Ausführungsbeispiele der Operateur eine Sternumshälfte anhebt, indem er die Länge der teleskopierbaren Stützstange 10 vergrößert, hat sich diese Stützstange immer zu dem Patienten P hin geneigt (sofern dieser ortsfest war), da die Länge zwischen dem Sternum und dem lösbaren Verbindungsmittel 30 zwischen Retraktorstange 20 und teleskopierbarer Stützstange 10 fest war. Der Ort des Sternums ist in diesem Fall die Mitte zwischen den beiden aufgespreizten Sternumshälften, also ungefähr der geometrische Mittelpunkt des Retraktors.

Wenn sich jetzt aber bei diesem Ausführungsbeispiel die teleskopierbare Stützstange 10 bei einer Verlängerung derselben nicht zu dem Patienten P hin neigen kann, würde der Patient P zu der teleskopierbaren Stützstange 10 hingezogen, was mit erheblichen Krafteinwirkungen auf den Patienten P einher ginge und zu traumatischen Verletzungen führen könnte. Daher ist bei diesem Ausführungsbeispiel der Retraktorstange 20 frei teleskopierbar ausgebildet, sodass keine seitlichen Kräfte auf den Patienten P einwirken. Der innere Teil 20B der Retraktorstange 20 kann dazu über einen ausreichend großen Bereich frei in dem äußeren Teil 20A der Retraktorstange 20 hin und her bewegt werden. Eine Verriegelung ist bei der teleskopierbaren Retraktorstange 20 nicht erforderlich, für eine sichere und Platz sparende Aufbewahrung kann sie aber vorteilhaft sein. Wenn bei dieser teleskopierbaren Retraktorstange 20B die Innenstange gegenüber der Außenstange 20A verdrehbar ist, kann auf diesem Weg auch die Verdrehung um den Winkel ß absorbiert werden.

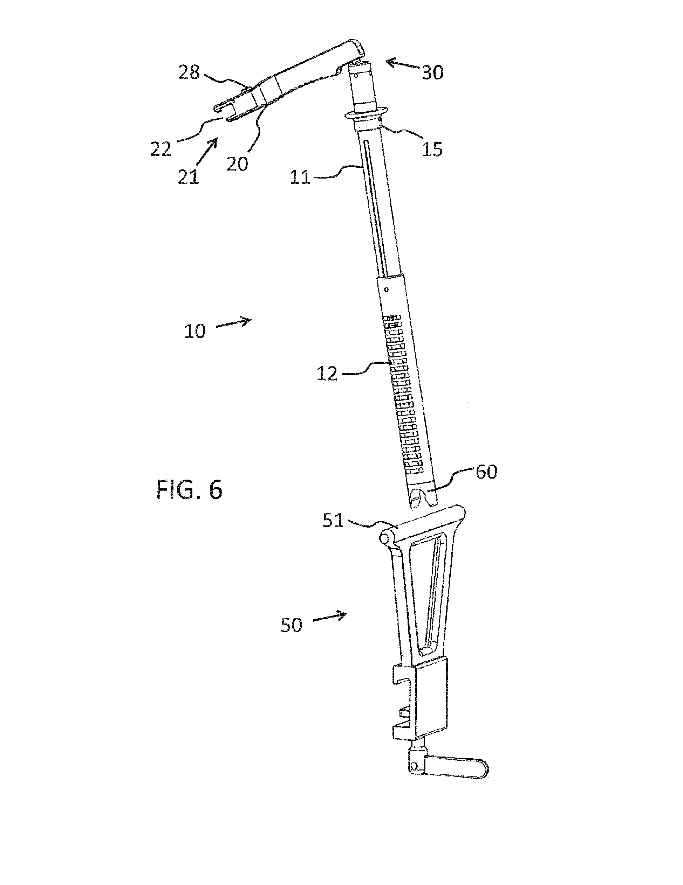

Im Folgenden ist ein sechstes Ausführungsbeispiel unter Bezugnahme auf die Fig. 6 und 8 bis 13 beschrieben.

Dieses sechste Ausführungsbeispiel zeigt eine teleskopierbare Retraktorhalterung mit einer teleskopierbaren Stützstange 10 mit einem Innenrohr 11, einem Außenrohr 12 und einem Verriegelungsmechanismus sowie einer Retraktorstange 20, die an ihrem einen Ende eine Retraktoraufnahme 21 aufweist. Die Retraktoraufnahme 21 ist daran angepasst, einen Retraktor R lösbar und formschlüssig zu greifen und ist identisch zu denen der vorangehenden Ausführungsbeispiele. Die Retraktorstange 20, die eine feste Länge hat, das heißt sie ist nicht teleskopierbar, ist an ihrem anderen Ende über eine lösbare Kugelgelenkverbindung 30 an dem Innenrohr 11 der teleskopierbaren Stützstange 10 befestigt. Ein Betätigungsring 15 durch den der Verriegelungsmechanismus von der Sperrstellung in die Freigabestellung überführt wird, ist knapp unterhalb des Kugelgelenks 30 an dem Innenrohr 11 der teleskopierbaren Stützstange 10 angebracht.

Die teleskopierbare Retraktorhalterung gemäß dem sechsten Ausführungsbeispiel weist einen Befestigungsmechanismus 50 auf, welcher eine Befestigungsschiene 51 hat und daran angepasst ist, lösbar an einer Operationstischschiene 100 eines Operationstischs 0 befestigt zu werden. Im vorliegenden Fall wird der Befestigungsmechanismus 50 seitlich auf die Operationstischschiene aufgesteckt und an dieser mit Hilfe einer Schraube verklemmt. Das Außenrohr 12 der teleskopierbaren Stützstange 10 weist eine Befestigungsaufnahme 60 auf, die auf die Befestigungsschiene 51 entlang der Längsrichtung der Befestigungsschiene 51 aufschiebbar ist.

Die Befestigungsschiene 51 weist einen Querschnitt auf, der aus einer im Wesentlichen kreisförmigen Fläche 52 und einer weiteren Fläche 53 zusammengesetzt ist, wobei die weitere Fläche eine Kreissegmentfläche ist, sodass beide Flächen zusammen Im Wesentlichen der Form eines Schlüssellochs entsprechen. Die Befestigungsaufnahme 60 der teleskopierbaren Stützstange 10 weist eine Aussparung 61 mit einer Innenwand 62 und zwei Seitenwänden 63 auf, wobei der Querschnitt der Aussparung 61 im Wesentlichen dem Querschnitt der Befestigungsschiene 51 entspricht also auch im Wesentlichen die Form eines Schlüssellochs hat. Zudem stellt die Innenwand 62 der Aussparung 61 mit der Befestigungsschiene 51 im aufgeschobenen Zustand mindestens eine Hinterschneidung her. Auf diese Weise können in der teleskopierbaren Stützstange 10 herrschende Zugkräfte von der Befestigungsaufnahme 60 auf die Befestigungsschiene 51 des Befestigungsmechanismus 50 übertragen werden. Die Seitenwände 63 der Aussparung 61 sind dabei so zu den Seitenflächen 54 der weiteren Fläche 53 der Querschnittsfläche der Befestigungsschiene 51 beabstandet und ausgerichtet, dass eine Verdrehung der teleskopierbaren Stützstange 10 relativ zu der Befestigungsschiene 51 auf einen vorbestimmten Winkelbereich begrenzt ist, wie dies insbesondere aus den Fig. 10 und 11 ersichtlich ist.

Bei den Winkeln und Abständen der Seitenflächen 54 der Befestigungsschiene 51 zu den Seitenwänden 63 der Befestigungsaussparung 60, wie sie in den Fig. 10 und 11 gezeigt sind, wird nicht nur ein Wegkippen der Retraktorhalterung verhindert, sondern es wird ebenfalls verhindert, dass die Retraktorhalterung auf den Patienten P fällt, Dies bedeutet, wenn die teleskopierbare Retraktorhalterung auf die Befestigungsschiene 51 des Befestigungsmechanismus 50 geschoben ist, kann sie sich nur noch in einem vorbestimmten Winkelbereich gegenüber der Befestigungsschiene 51 verdrehen. Sie kann so weder den Patienten P schädigen, indem sie auf ihn kippt bzw. fällt, noch kann sie aus dem sterilen Bereich in den unsterilen oder zumindest nicht sicher sterilen Bereich kippen bzw. fallen. Wenn zudem die Retraktoraufnahme 21 an dem Retraktor R befestigt ist, kann die teleskopierbare Stützstange 10 auch nicht mehr versehentlich wegrutschen.

An diesem Ausführungsbeispiel wird die Funktionsweise noch einmal unter Bezugnahme auf die Fig. 11 bis 13 detailliert erläutert. Hierbei gibt es bei dem vorliegenden Ausführungsbeispiel zwei verschiedene Vorgehensweisen.

Die erste Vorgehensweise ist den zuvor beschriebenen Vorgehensweisen recht ähnlich. Hierzu weist die teleskopierbare Retraktorhalterung zwei Befestigungsmechanismen 50 auf, von denen je einer vor Beginn der Operation auf jeder Seite des Operationstisches an den Operationstischschienen 100 befestigt wird.

Der Operateur öffnet zunächst das Sternum des Patienten P, setzt den Retraktor R ein und spreizt mit Hilfe des Retraktors R das Sternum des Patienten P. Um eine Seite des Sternums zu exponieren, das heißt anzuheben, um beispielsweise eine an der Brustinnenwand verlaufende Arterie frei zu präparieren, schiebt der Arzt die teleskopierbare Retraktorhalterung auf der Seite der anzuhebenden Sternumshälfte auf die Befestigungsschiene 51 auf. Anschließend fixiert er mit Hilfe der Retraktoraufnahme 21 den Retraktor R an der Retraktorhalterung. Wenn der Operateur nun die Seite des Sternums anheben möchte, auf der sich die Retraktorhalterung befindet, verlängert er über den Einhand-Betätigungsmechanismus 15 die teleskopierbare Stützstange 10, wodurch sich die Retraktorstange 20 mitsamt dem Retraktor R neigt. Dabei stützt sich der distale Schenkel des Retraktors R auf der nicht anzuhebenden Sternumshälfte auf und der proximale Schenkel des Retraktors R zieht die anzuhebende Sternumshälfte nach oben.

Nach erfolgreicher Arbeit auf dieser Seite des Patienten P wird die teleskopierbare Stützstange 10 so weit verkürzt, dass die Retraktoraufnahme 21 weitestgehend momentenfrei ist. In diesem Zustand lässt sich der Retraktor R besonders leicht und mit einer einzigen Hand vom Retraktor R lösen. Der Operateur löst also den Retraktor R von der Retraktoraufnahme 21 und schiebt bzw. zieht die teleskopierbare Retraktorhalterung seitlich in Längsrichtung der Befestigungsschiene 51 von dem Befestigungsmechanismus 50. Anschließend schiebt er, ohne umgreifen zu müssen, die teleskopierbare Retraktorhalterung auf die gegenüber vom Patienten P angebrachte Befestigungsschiene 51 des zweiten Befestigungsmechanismus 50, bringt die Retraktoraufnahme 21 an dem Retraktors R, das heißt am gegenüberliegenden Schenkel, wie zuvor, an und kann nun wieder mit Hilfe einer Verlängerung der teleskopierbaren Stützstange 10 eine Hälfte des Sternums anheben, nämlich nun die andere Hälfte des Sternums.

Auf diese Weise kann ein Operateur ohne Hilfspersonal nacheinander die gewünschte Sternumshälfte exponieren bzw. anheben, ohne dabei der Gefahr ausgesetzt zu sein, unsteril zu werden. Bei dieser ersten Vorgehensweise hat der Operateur den Vorteil, dass die teleskopierbare Retraktorhalterung immer auf der Seite des Patienten P angeordnet ist, auf der das Sternum gerade angehoben wird. Sinnvollerweise befindet sich der Operateur immer auf der Seite der nicht angehobenen Sternumshälfte, da er von dort die beste Sicht auf den zu präparierenden Bereich im Brustraum des Patienten P hat.

Bei der zweiten Vorgehensweise benötigt der Operateur nur einen einzigen Befestigungsmechanismus 50, der vor Beginn der Operation auf nur einer Seite des Operationstischs 0 an einer Operationstischschiene 100 befestigt wird.

Zunächst macht der Operateur alles wie bei der ersten Vorgehensweise, d.h. er fixiert die teleskopierbare Retraktorhalterung an dem Befestigungsmechanismus 50, befestigt den Retraktor R an der Retraktoraufnahme 21 und verlängert die teleskopierbare Stützstange 10, um die Hälfte des Sternums anzuheben, auf deren Seite die Retraktorhalterung angeordnet ist. Will der Operateur nun die andere Sternumshälfte anheben, muss er die teleskopierbare Retraktorhalterung gar nicht auf die andere Seite des Patienten P umbauen, sondern er kann die teleskopierbare Stützstange 10 einfach über den im Wesentlichen momentenfreien Zustand der Retraktoraufnahme 21 hinaus verkürzen. Dabei wird ein Druck auf die Sternumshälfte aufgebracht, auf deren Seite sich die Retraktorhalterung befindet, Da sich, wie oben bereits beschrieben, Rippen schwerer eindrücken als aufbiegen lassen, verbleibt die gedrückte Sternumshälfte im Wesentlichen in ihrer Ausgangsposition (zwar gespreizt, aber nicht verdreht bzw. angehoben oder abgesenkt) und die andere Sternumshälfte wird angehoben bzw. exponiert. In diesem Fall befindet sich die teleskopierbare Retraktorhalterung zwar auf der Seite, auf der sich auch der Operateur befindet, nachdem die teleskopierbare Stützstange 10 aber stark verkürzt ist, stört sie den Operateur bei der Operation nicht. Die auf den Patienten P ausgeübten Kräfte sind bei einem Exponieren einer Sternumshälfte durch Verkürzung der teleskopierbaren Retraktorhalterung zwar geringfügig größer als beim Exponieren durch Verlängerung der teleskopierbaren Retraktorhalterung, aber für die meisten Patienten P spielt dies keine Rolle. Mit dieser zweiten Vorgehensweise braucht der Operateur die teleskopierbare Retraktorhalterung nicht umbauen und spart somit sowohl einen Befestigungsmechanismus 50 sowie die Zeit für den Umbau.

Im Folgenden ist ein siebtes Ausführungsbeispiel unter Bezugnahme auf die Fig. 7 beschrieben. Das siebte Ausführungsbeispiel unterscheidet sich von dem sechsten Ausführungsbeispiel dadurch, dass die Befestigungsaufnahme 60 nicht am freien Ende der teleskopierbaren Stützstange 10 angeordnet ist, sondern seitlich an der teleskopierbaren Stützstange 10, genauer gesagt an dem Außenrohr 12, angebracht ist. Außerdem ist zwischen der Retraktrostange 20 und der teleskopierbaren Stützstange 10 anstelle eines Kugelgelenks ein lösbares Scharniergelenk 30, beispielsweise mit einem leicht abziehbaren Splintbolzen vorgesehen.

Im Folgenden sind noch weitere Vorteile einzelner Ausführungsbeispiele dargestellt.

Die Verwendung einer oder mehrerer Befestigungsmechanismen 50 hat auch den Vorteil, dass der Operateur mit der Befestigungsschiene 51 kein Risiko eingeht, anderes Operationsmaterial zu beschädigen. Denn würde der Operateur die teleskopierbare Retraktorhalterung direkt auf der abgedeckten Operationstischschiene abstützen, könnte er nicht wissen, ob seine Abstützstelle frei von Kabeln, Schläuchen oder Lichtleitern ist, welche sich unter den Tüchern verbergen können, die den Operationstisch 0 bedecken, und welche er abklemmen könnte.

In der Fig. 10 ist die Geometrie einer Befestigungsaufnahme 60 und einer Befestigungsschiene 51 gezeigt. Hierbei bilden die Vorsprünge, die an den Übergängen der Innenwand 62 zu den Seitenwänden 63, 63 ausgebildet sind, Hinterschneidungen mit dem Übergang der Mantelfläche 55 zu den Seitenflächen 54, 54 der Befestigungsschiene 51. Wie man in Fig. 10 erkennen kann, sind die Seitenflächen 54, 54 parallel zueinander, während die Seitenwände 63, 63 einen gewissen Winkel zwischen sich aufspannen. Auf diese Weise besteht eine gewisse Bewegungsfreiheit der teleskopierbaren Stützstange 10 um die Befestigungsschiene 51 herum, die eine Anpassung an die veränderliche Geometrie der teleskopierbaren Retraktorhalterung bei der Verwendung zulässt. Gleichzeitig begrenzen die Seitenwände 63, 63 eine Verdrehung der teleskopierbaren Stützstange 10 um die Befestigungsschiene 51 herum, sodass der obere Bereich der telekopierbaren Stützstange 10 nicht in den unsterilen Bereich kippen kann und auch nicht auf den Patienten P kippen kann. Die Winkel der beiden Seitenwände 63, 63 müssen nicht symmetrisch zur Längsachse der teleskopierbaren Stützstange 10 sein, sondern können nach Bedarf festgelegt werden.

Um ein seitliches Aufschieben der Befestigungsaufnahme 60 auf die Befestigungsschiene 51 zu erleichtern, kann die Befestigungsschiene an einem oder an beiden Enden verjüngt sein. Dies erleichtert das Aufschieben insbesondere im Einhandbetrieb. Außerdem wird der Operateur dadurch nicht in die Versuchung geführt, die teleskopierbare Stützstange 10 im Bereich der Befestigungsaufnahme 60, also im unsterilen Bereich, anzufassen.

Die Befestigungsaufnahme muss auch nicht zum freien Ende der teleskopierbaren Stützstange 10 hin geöffnet sein, sondern kann in einem beliebigen Winkel zur Längsachse der teleskopierbaren Stützstange 10 angeordnet sein. Beispielsweise kann sich die Befestigungsaufnahme 60 quer zur Längsachse der teleskopierbaren Stützstange 10 zum Operationstisch 0 hin öffnen und die Befestigungsschiene kann um 90° gedreht von dem Operationstisch 0 wegzeigend angeordnet sein.

Außerdem kann statt einem zweiteiligen Teleskopmechanismus ein dreiteiliger oder mehrteiliger Teleskopmechanismus in der teleskopierbaren Retraktorhalterung verwendet werden. Das Innenrohr 11 kann gegenüber dem Außenrohr 12 unverdrehbar sein, aber auch in einem gewissen Bereich verdrehbar.

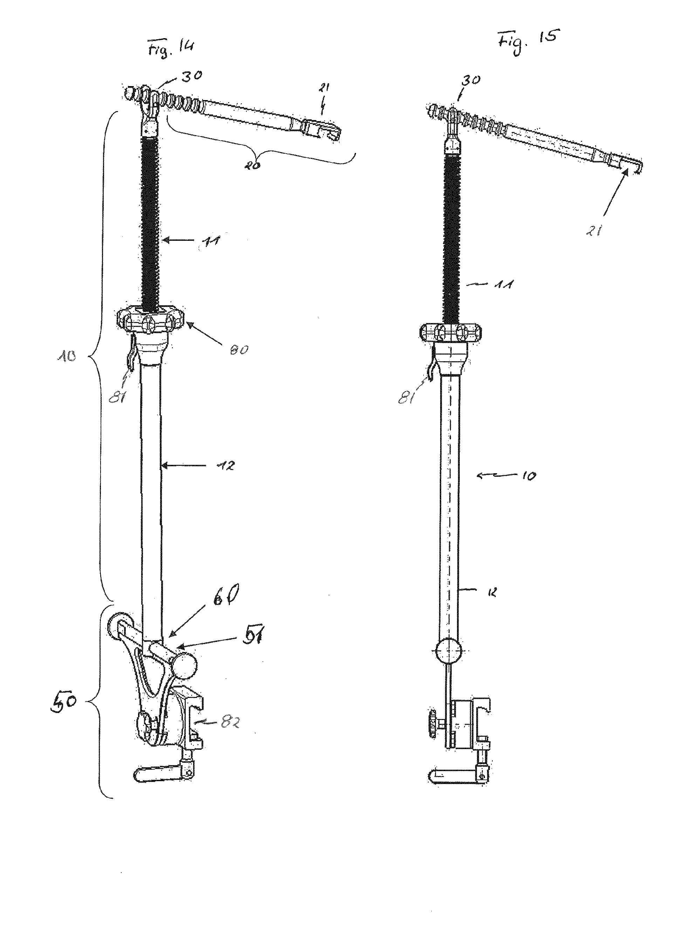

In den Fig. 14 - 19 ist ein achtes, erfindungsgemäßes Ausführungsbeispiel einer Retraktorhalterung dargestellt. Im Unterschied zu den vorstehend genannten Ausführungsbeispielen ist hier die Wirkverbindung zwischen der Retraktorstange (Retraktorhebel) 20 und der Stützstange (Stütze) 10 nicht durch ein lösbares Scharnier- oder Kugelgelenk sondern durch einen losen Verhakmechanismus realisiert.

Im Einzelnen besteht die Stützstange 10 gemäß der Fig. 14 und 15 aus dem Innenrohr 11 nunmehr in Form einer Spindel, das in dem Außenrohr 12 in Form eines Aufnahmezylinders axialbeweglich sowie drehbar gelagert ist. Auf die Spindel 11 ist eine Handmutter 80 aufgedreht, die sich an einer Stirnseite des Aufnahmezylinders 12 axial abstützt. Ferner kann an dem Aufnahmezylinder 12 noch eine weitere manuell, beispielsweise über einen Hebel 81 lösbare Verrastung (nicht weiter gezeigt) vorgesehen sein, mittels der die Spindel 11 schnell für ein Herausnehmen aus dem Aufnahmezylinder 12 freigegeben werden kann, etwa um die einzelnen Segmente der Stütze 10 reinigen zu können.

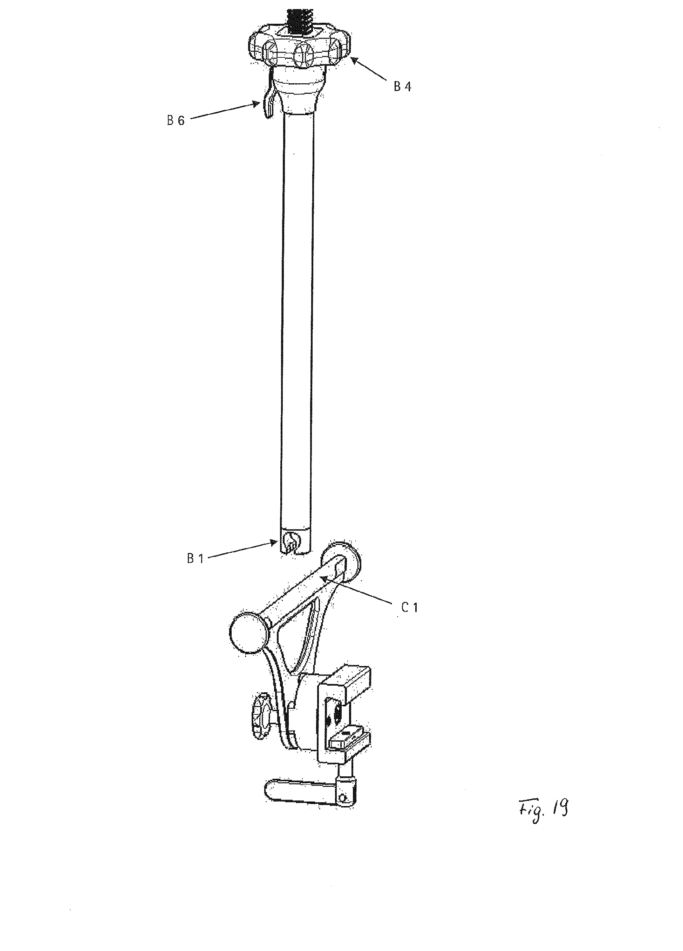

An dem einen (unteren) Ende der Stützstange 10 ist der Befestigungsmechanismus 50 zur Fixierung der teleskopierbaren Retraktorhalterung an einem Operationstisch oder dergleichen Unterbau vorgesehen, wie dies insbesondere in Fig. 19 dargestellt ist. Wie vorstehend bereits beschrieben, hat der Befestigungsmechanismus 50 als eigenständige, separate Einheit eine manuell betätigbare Klemme oder Schraubzwinge 82 zur Fixierug am OP-Tisch, an der vorzugsweise schwenk-/drehbar und arretierbar eine Befestigungsschiene 51 montiert ist. Im Gegenzug hat die Stützstange 10 eine Befestigungsaufnahme 60 an ihrem unteren Ende oder Endabschnitt, die mit der Befestigungsschiene 51 in vorzugsweise scharnierförmigen Eingriff bringbar ist.

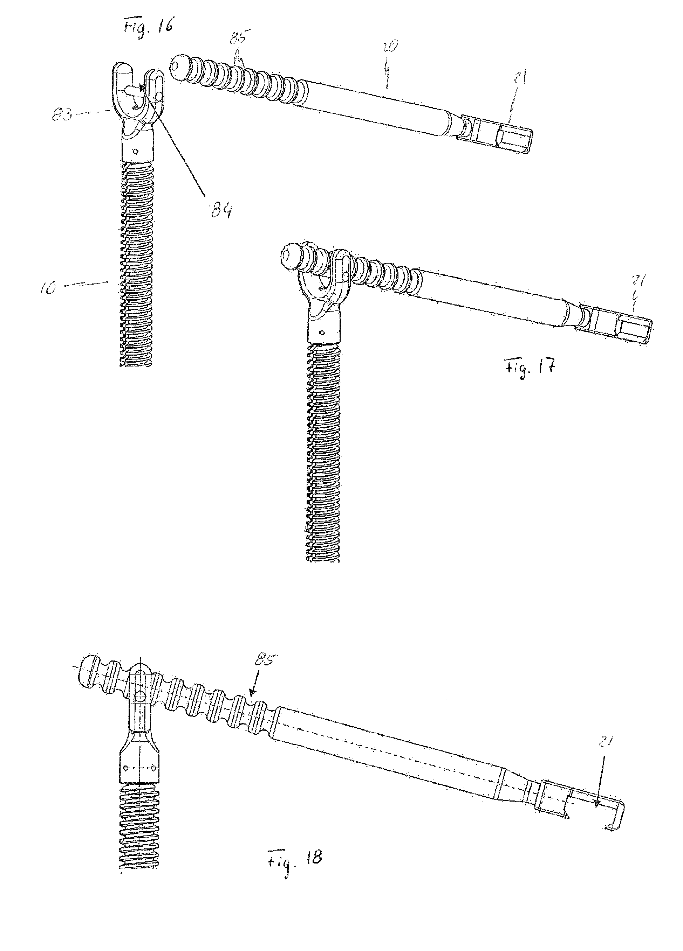

Wie insbesondere in den Fig. 16 - 18 dargestellt wird, ist an dem gegenüberliegenden (oberen) Ende der Stützstange 10 ein U- oder gabelförmiges Auflagerelement 83 angeordnet, derart, dass sich die beiden Schenkel des Auflagerelements 83 parallel zueinander sowie axial zur Stützstange 10 erstrecken. Die beiden Schenkel sind durch einen Querbolzen 84 miteinander verbunden. Alternativ hierzu ist es aber auch denkbar, am oberen Ende der Stützstange eine Querkante oder Leiste anzuformen, welche dann dem Querbolzen entspräche und die Querkante an ihren jeweiligen Kanten-Längsenden mit Endanschlägen zu versehen oder auszubilden.

Wie ferner insbesondere in den Fig. 16 - 18 gezeigt ist, hat die Retraktorstange 20 an ihrem proximalen Endabschnitt eine Anzahl von axial beabstandeten Ein-/Angriffsstellen 85 in Form von Wellenringen oder Nuten, wodurch eine entsprechende Anzahl von (radialen) Hinterschneidungen gebildet werden. In anderen Worten ausgedrückt, sind in der Retraktorstange 20 in diesem Ausführungsbeispiel keine Lageraugen ausgebildet, sondern radial frei zugänglich Hinterschneidungen, in die der Querbolzen oder Querleiste 84 an der Stützstange 10 in Querrichtung des Bolzens/Leiste 84 einführbar ist. Dabei sei darauf hingewiesen, dass an der Retraktorstange 20 auch eine Art Lagerauge ausgebildet sein kann, das jedoch klammerförmig in Umfangsrichtung nicht geschlossen sondern geschlitzt ist, sodass auch in diesem Fall der Querbolzen oder Querleiste 84 in deren Querrichtung einrastbar/einclipbar ist.

Insoweit bewirken die Hinterschneidungen 85 eine Verhinderung eines Längsabrutschens der Retraktorstange 20 vom Querbolzen 84 an der ausgewählten Angriffsstelle und die beiden Schenkel ein Querabrutschen der Retraktorstange 20 vom Bolzen 84. Des Weiteren erlaubt die Hinterschneidung auch weiterhin ein scharnierförmiges Verschwenken der Retraktorstange 20 um den Querbolzen 84, wobei jedoch die Wirkverbindung zwischen den beiden Bauteilen Retraktorstange 20 und Stützstange 10 durch eine freie/lose Auflagerung bewirkt wird.

Das Gesamtsystem der erfindungsgemäßen Hub-Vorrichtung, d.h. der teleskopierbaren Retraktorhalterung besteht folglich grundlegend aus den drei verschiedenen separaten, d.h. lösbar miteinander verbundenen/verbindbaren Hauptbestandteilen:
Zum einen aus einem aus dem Retraktorhebel 20 mit Adaptionseinrichtung 21 für den Retraktor bestehenden Teil, einem zweiten Teil, nämlich der sogenannten Stütze 10, welche eine höhenverstellbare Vorrichtung darstellt, und einem dritten Teil, welcher den Adapter (Befestigungsmechanismus) 50 zur 0P- Tisch-Schiene bildet, auf dem die Stütze 10 aufgesetzt wird.

Schon vor der Operation beim Vorbereiten des OP-Equipments wird der OP-Tisch mit sterilen Tüchern abgedeckt. Dann wird darüber der Tischadapter (Befestigungmechanismus) 50 an der OP-Tisch-Schiene befestigt, wodurch die Problematik des sterilen An- und Umbaus umgangen wird. Der Adapter 50 selbst ist nun steril und die Stütze 10, die darauf aufgesetzt wird, ragt nicht in den unsterilen Bereich unterhalb der OP-Tisch-Ebene. Falls beide Mammaria-Arterien im Brustraum des Patienten prepariert werden sollen, werden auf beiden Seiten des OP-Tisches je ein Adapter 50 angebracht. Die Stütze 10 kann dann steril von einem auf den anderen Adapter 50 durch lösen der Klemme 82 umgesetzt werden.

Dann beginnt die Operation; anfangs ohne Verwendung von Retraktorhebel 20 oder Stütze 10, mit dem Durchtrennen des Sternums und dem Einsetzen des Retraktors. Für die Präparation der Mammaria-Arterien wird nun vom Operateur die Stütze 10 auf der ihm gegenüberliegenden Tisch-Seite auf den dortigen Tischadapter 50 an dessen Befestigungsschiene 51 aufgesetzt. Dann adaptiert er den Retraktorhebel 20 an dessen Retraktoraufnahme 21 mit dem Retraktor, derart, dass das freie proximale Ende des Retraktorhebels 20 in Richtung hin zur Stütze 10 zeigt. Beide genannten Arbeitsgänge funktionieren nur durch einfaches Aufstecken, beziehungsweise Aufschieben der Retraktoraufnahme 21 auf den Retraktor, wie dies vorstehend bereits geschildert wurde.

Dadurch, dass der Tischadapter 50 im vorliegenden Ausführungsbeispiel selbst über die Befestigungsschiene 51 verfügt, die oberhalb und parallel zur Tisch-Schiene verläuft bzw. ausgerichtet werden kann, hat der Operateur genug Spielraum, um den Längs-Versatz zwischen dem Tischadapter 50 und dem Retraktor, der aus der Größe und Lage des Patienten resultiert, auszugleichen. Diese Schiene 51 richtet im Zusammenspiel mit der formschlüssigen Adaptionsstelle/Befestiungsaufnahme 60 die Stütze 10 möglichst vertikal aus, sodass sie über einen festen Stand verfügt und nicht in den Operationsbereich fallen kann. Ein weiterer Vorteil besteht darin, dass der Operateur mit der Schiene 51 als Adaptionsstelle kein Risiko eingeht, anderes Operationsmaterial zu beschädigen.

Nach der Konnektion von Retaktorhebel 20 mit Retraktor und Stütze 10 mit Tischadapter 50 wird die Stütze 10 mittels des Betätigungselements / Handrad / Handmutter 80 um ein gewisses Maß in der Höhe nach oben verstellt. Im vorliegenden Ausführungsbeispiel geschieht dies mittels der Gewindestange 11 in dem Teleskoprohr 12 und der Mutter 80, die als relativ großes Betätigungselement ausgebildet ist. Mit dem Retraktorhebel 20, der nachfolgend lose auf dem Querbolzen 84 an einer ausgewählten Angriffsstelle 85 vertikal aufgelegt wird, kann der Retraktor einseitig angehoben werden - dank des physikalisch guten Hebelverhältnisses gestaltet sich dies sehr leichtgängig. Das freie, proximale Ende des Retraktorhebels 20 wird dann am oberen Ende der Stütze 10 in einer vorgesehenen Angriffsstelle 85 am Querbolzen / Querleiste 85 eingelegt, wobei die Angriffsstelle 85 hinterschneidend so ausgebildet ist, dass der Retraktorhebel 20 nicht auf die Seite sowie in Längsrichtung vom Querbolzen 85 abrutschen kann. Nun kann die Stütze 10 noch weiter zu deren Längenvergrößerung verstellt werden, was aufgrund des Spindelantriebs wesentlich feinfühliger als das bloße Anheben von Hand vonstatten geht, bis für den Operateur optimale Sichtverhältnisse zur Arterie vorliegen. Die Stütze 10 des Retraktors steht dann schräg in Bezug zu ihrer Ausgangslage.

Ist der Operateur mit diesem System vertraut, kann ein nachträgliches Verstellen entfallen, da schon vor der Verbindung des Retraktorhebels 20 mit der Stütze 10 die passende Höhe der Stütze 10 voreingestellt wurde.

Eine weitere Möglichkeit der Anwendung des Systems besteht darin, den Retraktorhebel 20 auf eine noch nicht höhenverstellte, sich in ihrer Minimalhöhe befindliche Stütze aufzusetzen/aufzulegen und dann nur mittels des Betätigungselements 80 die Schrägstellung des Retraktors hervorzurufen. Durch die erfindungsgemäße Trennung von Stütze 10 und Retraktorhebel 20 in zwei Teile so wie die lose/lösbare Verbindung dazwischen kann der Operateur also sehr flexibel und individuell arbeiten.

Damit das Anheben und das anschließende Halten dieser exponierten Stellung des Sternums möglichst atraumatisch für den Patienten geschehen, befindet sich zwischen dem Retraktorhebel 20 und der Stütze 10 keine feste, arretierbare oder formschlüssige Verbindung. Somit kann zwischen dem Retraktorhebel 20 und Stütze 10 in jeder Ebene ein gewisser Winkelversatz ausgeglichen werden, ohne dass es zu negativen Auswirkungen auf den Patienten kommt. Die Längsachse der Stütze 10 steht immer im Wesentlichen vertikal, wodurch kein Platz außerhalb der OP-Tischfläche beansprucht wird. Die Stütze 10 baut zudem nicht wesentlich höher als der schräg stehende Retraktor, was ein weiterer Vorteil gegenüber den anderen Systemen mit externer Befestigung darstellt.

In der beschriebenen Ausführungsvariante gemäß dem achten, erfindungsgemäßen Ausführungsbeispiel könnte das Spindelgewinde zur Höhenverstellung auch durch eine Zahnstange und einen dementsprechenden Kurbelantrieb ersetzt werden. Weiterhin könnte die Stütze 10 wie bei den zuvor beschriebenen Ausführungsbeispielen als mehrteiliges Teleskop ausgebildet werden, um eine Hubhöhe zu schaffen, die größer als das erste Einzel-Element ist. Damit könnten alle Elemente einerseits sehr kompakt ineinander zusammengeschoben und andererseits auf eine große Höhe ausgezogen werden.

Zwischen den einzelnen Segmenten 11, 12 der höhenverstellbaren Stütze 10 kann wie vorstehend bereits angedeutet wurde optional ein kleines Betätigungselement 81 vorgesehen werden, um die Segmente 11, 12 auseinanderzubauen und besser reinigen zu können. Im zusammengebauten Zustand wird mit diesem Element 81 verhindert, dass der Anwender die Segmente 11, 12 versehentlich auseinanderzieht. Eine weitere Ausführungsvariante könnte schließlich auf den gesamten Tischadapter 50 verzichten und die Stütze 10 direkt an die OP-Tisch-Schiene oder auf andere Haltevorrichtungen im Operationsgebiet aufsetzen.

Der Retraktionshebel 20 kann an seinem proximalen Ende als ergonomischer Handgriff ausgebildet sein (mit einer oder mehreren Angriffsstellen 85 am freien Ende des Griffs oder nur die Angriffsstellen (Rastpositionen) 85 zum Einlegen in das Gegenstück (Querbolzen 84) der Stütze 10 aufweisen.

Der Retraktorhebel 20 verfügt folglich idealerweise über keinerlei Befestigungs- oder Betätigungselemente, sondern kann rein formschlüssig mit der Gegenform eines Refraktors zusammengefügt werden, beispielsweise durch einfaches Aufschieben oder Stecken

Weitere Kombinationen der einzelnen Merkmale sind möglich und dem Fachmann ergeben sich aus dieser Beschreibung und den beigefügten Ansprüchen und Figuren zahlreiche weitere Modifikationen und Abwandlungen.

## Patentansprüche

1. Teleskopierbare Retraktorhalterung
mit einer längenverstellbaren Stütze (10), die an ihrem einen Endabschnitt an einem Operationstisch (O) abstützbar ist, und
mit einem Retraktorhebel (20), der an seinem einen, distalen Ende eine Retraktoraufnahme (21) aufweist, welche daran angepasst ist, einen Retraktor (R) lösbar zu greifen,
wobei der Retraktorhebel an seinem anderen, proximalen Endabschnitt mit dem anderen freien Endabschnitt der Stütze (10) über ein lösbares Verbindungsmittel für eine Hebelkraftbeaufschlagung stützend sowie lösbar wirkverbindbar ist, wobei der Retraktorhebel (20) an seinem proximalen Endabschnitt zumindest eine und vorzugsweise mehrere längsbeabstandete Ein-/Angriffsstellen (85) als das Verbindungsmittel aufweist,
**dadurch gekennzeichnet, dass**
die Stütze (10) teleskopierbar ist und der Retraktorhebel (20) an der zumindest einen Ein-/Angriffsstelle (85) mit der Stütze (10) in lose aufliegende Wirkverbindung wahlweise bringbar ist,
die zumindest eine Ein-/Angriffsstelle (85) eine frei zugängliche Hinterschneidung ausbildet, in die ein Rastvorsprung oder Bolzen (84) an dem anderen freien Endabschnitt der Stütze (10) einrastbar ist, derart, dass eine relative Schwenkbewegung zwischen Stütze (10) und Retraktorhebel (20) zugelassen wird, ein Abgleiten des Retraktorhebels (20) in dessen Längsrichtung vom der Stütze (10) jedoch behindert wird, und
die Hinterschneidung (85) in Form einer Einkerbung oder eines Vorsprungs am stangenförmigen Retraktorhebel (20) gebildet ist und mit dem Rastvorsprung oder Bolzen am freien Ende der Stütze (10) durch einfache Auflage zusammenwirkt.

2. Teleskopierbare Retraktorhalterung nach Anspruch 1 oder 2, **dadurch gekennzeichnet, dass** die längenverstellbare Stütze (10) ein teleskopierbares Stützrohr mit einem Innenrohr (11) und einem Außenrohr (12) ist, die über eine Handhabe (80) relativ bewegbar und in einer gewählten Längeneinstellung gegeneinander arretierbar sind.

3. Teleskopierbare Retraktorhalterung nach Anspruch 2, **dadurch gekennzeichnet, dass** das Stützrohr (10) als ein Spindelmechanismus vorzugsweise mit dem Innenrohr (11) als Spindel oder Gewindestange ausgebildet ist, die in dem Außenrohr (12) als Teleskoprohr geführt ist und die Handhabe (80) ein Handrad oder eine Schraubenmutter aufweist, die auf die Spindel (11) aufgedreht ist und sich gegen das Teleskoprohr (12) axial abstützt.

4. Teleskopierbare Retraktorhalterung nach einem der vorstehenden Ansprüche, **dadurch gekennzeichnet, dass** der Retraktorhebel (20) eine Retraktorstange ist.

5. Teleskopierbare Retraktorhalterung nach einem der vorstehenden Ansprüche, **dadurch gekennzeichnet, dass** sich die Einkerbung oder der Vorsprung umlaufend am stangenförmigen Retraktorhebel (20) ausbildet.

6. Teleskopierbare Retraktorhalterung nach Anspruch 1, **dadurch gekennzeichnet, dass** die teleskopierbare Stütze (10) gelenkig an einem Operationstisch (O) abstützbar ist.

7. Teleskopierbare Retraktorhalterung nach einem der vorstehenden Ansprüche, **gekennzeichnet durch** einen separaten adaptiven Befestigungsmechanismus (50), der zum Fixieren an einem Operationstisch (O) oder dergleichen Unterbau speziell oder universal angepasst ist und eine eigene Befestigungsschiene (51) aufweist, an welche die Stütze (10) abstützend ankoppelbar ist.

8. Teleskopierbare Retraktorhalterung nach Anspruch 7, **dadurch gekennzeichnet, dass** die Stütze (1O) eine Befestigungsaufnahme (60) hat, die mit der Befestigungsschiene (51) scharnierartig in Kopplungseingriff bringbar ist.

## Claims

1. A telescoping retractor holder
comprising a length-adjustable support (10), the one end section of which can be supported on an operating table (O), and comprising a retractor lever (20), the one, distal end of which comprises a retractor receptacle (21) adapted to releasably grip a retractor (R),
and the other, proximal end section of which can be operatively connected in a supporting and detachable manner to the other free end section of the telescoping support (10), by way of a detachable connecting means, for the application of a lever force, wherein
the proximal end section of the retractor lever (20) comprising at least one, and preferably a plurality of longitudinally spaced points of engagement/points of action (85) serving as the connecting means,
**characterized in that**
the support (10) can be telescoped and the retractor lever (20) can selectively be operatively connected to the support (10) in a loosely seated manner at the at least one point of engagement/point of action (85),
the at least one point of engagement/point of action (85) forming a freely accessible undercut, into which a detent protrusion or pin (84) at the other free end section of the support (10) can latch in such a way that a relative pivoting movement is permitted between the support (10) and the retractor lever (20), while sliding of the retractor lever (20) in the longitudinal direction thereof away from the support (10) is prevented, and
the undercut (85) is designed in the form of a notch or a protrusion on the rod-shaped retractor lever (20), and interacts with the detent protrusion or pin at the free end of the support (10) by simple abutment.

2. The telescoping retractor holder according to claim 1 or 2, **characterized in that** the length-adjustable support (10) is a telescoping support pipe having an inner pipe (11) and an outer pipe (12), which can be moved relative to each other by way of a handle (80) and locked with respect to each other in a selected length setting.

3. The telescoping retractor holder according to claim 2, **characterized in that** the support pipe (10) is designed as a spindle mechanism, preferably with the inner pipe (11) designed as a spindle or threaded rod, which is guided in the outer pipe (12) serving as the telescoping pipe, and the handle (80) comprises a hand wheel or a screw nut, which is screwed onto the spindle (11) and is axially supported on the telescoping pipe (12).

4. The telescoping retractor holder according to one of the preceding claims, **characterized in that** the retractor lever (20) is a retractor rod.

5. The telescoping retractor holder according to one of the preceding claims, **characterized in that** the notch or the protrusion is formed around the periphery of the rod-shaped retractor lever (20).

6. The telescoping retractor holder according to according to claim 1, **characterized in that** the telescoping support (10) can be supported on an operating table (O) in an articulated manner.

7. The telescoping retractor holder according to one of the preceding claims, **characterized by** a separate adaptive fastening mechanism (50), which is specially or universally adapted for fixation to an operating table (O) or similar platform and comprises a dedicated fastening rail (51), to which the support (10) can be coupled in a supporting manner.

8. The telescoping retractor holder according to claim 7, **characterized in that** the support (10) has a fastening receptacle (60), which can be couplingly engaged in the fastening rail (51) in a hinge-like manner.

## Revendications

1. Fixation d'écarteur télescopique,
comprenant un montant (10) réglable en longueur, qui peut prendre appui, au niveau de l'une de ses sections d'extrémité, au niveau d'une table chirurgicale (O), et
comprenant un levier d'écarteur (20), qui présente, au niveau de l'une de ses extrémités distales, un logement d'écarteur (21), qui est adapté pour saisir de manière amovible un écarteur (R),
dans laquelle le levier d'écarteur peut être relié en liaison fonctionnelle, par un support et de manière amovible également, au niveau de son autre section d'extrémité proximale, à l'autre section d'extrémité libre du montant (10), par l'intermédiaire d'un moyen de liaison amovible en vue d'une application de force de levier,
dans laquelle le levier d'écarteur (20) présente, en tant que moyen de liaison, au niveau de sa section d'extrémité proximale, au moins un et de préférence plusieurs emplacements de prise/d'engagement (85) tenus à distance en longueur,
**caractérisée en ce**
**que** le montant (10) est télescopique, et que le levier d'écarteur (20) peut être amené au choix dans une liaison fonctionnelle mobile avec le montant (10) au niveau de l'au moins un emplacement de prise/d'engagement (85),
**que** l'au moins un emplacement de prise/d'engagement (85) réalise une contre-dépouille librement accessible, dans laquelle une partie faisant saillie d'enclenchement ou un boulon (84) peut être enclenché(e) au niveau de l'autre section d'extrémité libre du montant (10) de telle manière qu'un déplacement par pivotement relatif entre le montant (10) et le levier d'écarteur (20) est autorisé, qu'un détachement par glissement du levier d'écarteur (20) dans sa direction longitudinale du montant (10) est toutefois empêché, et
**que** la contre-dépouille (85) est formée sous la forme d'une encoche ou d'une partie faisant saillie au niveau du levier d'écarteur (20) présentant une forme de tige et coopère avec la partie faisant saillie d'enclenchement ou le boulon au niveau de l'extrémité libre du montant (10) par un appui simple.

2. Fixation d'écarteur télescopique selon la revendication 1 ou 2, **caractérisée en ce que** le montant (10) réglable en longueur est un tube support télescopique pourvu d'un tube intérieur (11) et d'un tube extérieur (12), qui peuvent être déplacés par l'intermédiaire d'une poignée (80) et qui peuvent être bloqués l'un contre l'autre dans une position en longueur choisie.

3. Fixation d'écarteur télescopique selon la revendication 2, **caractérisée en ce que** le tube support (10) faisant office de mécanisme à broche est réalisé de préférence avec le tube intérieur (11) sous la forme d'une broche ou d'une tige filetée, qui est guidée dans le tube extérieur (12) sous la forme d'un tube télescopique, et **en ce que** la poignée (80) présente une molette ou un écrou de vis, qui est tourné sur la broche (11) et qui s'appuie de manière axiale contre le tube télescopique (12).

4. Fixation d'écarteur télescopique selon l'une quelconque des revendications précédentes, **caractérisée en ce que** le levier d'écarteur (20) est une tige d'écarteur.

5. Fixation d'écarteur télescopique selon l'une quelconque des revendications précédentes, **caractérisée en ce que** l'encoche ou la parte faisant saillie se forme de manière périphérique au niveau du levier d'écarteur (20) présentant une forme de tige.

6. Fixation d'écarteur télescopique selon la revendication 1, **caractérisée en ce que** le montant (10) télescopique prend appui de manière articulée au niveau d'une table chirurgicale (O).

7. Fixation d'écarteur télescopique selon l'une quelconque des revendications précédentes, **caractérisée par** un mécanisme de fixation (50) adaptatif séparé, qui est adapté de manière spécifique ou de manière universelle afin d'être fixé au niveau d'une table chirurgicale (O) ou d'une sous-structure similaire ou qui présente un rail de fixation (51) propre, auquel le montant (10) peut être couplé en appui.

8. Fixation d'écarteur télescopique selon la revendication 7, **caractérisée en ce que** le montant (10) a un logement de fixation (60), qui peut être amené en prise par couplage avec le rail de fixation (51) à la manière d'une charnière.
